(19) Europäisches Patentamt

European Patent Office

Office européen des brevets

(11) EP 1 649 928 A1

(12) EUROPEAN PATENT APPLICATION
published in accordance with Art. 158(3) EPC

(43) Date of publication:
26.04.2006 Bulletin 2006/17

(51) Int Cl.:
B01J 20/26 (1980.01)     B32B 5/30 (1968.09)

(21) Application number: 04736139.9

(22) Date of filing: 04.06.2004

(86) International application number:
PCT/JP2004/008177

(87) International publication number:
WO 2004/108274 (16.12.2004 Gazette 2004/51)

(84) Designated Contracting States:
DE

(30) Priority: 06.06.2003 JP 2003162077
06.06.2003 JP 2003162078
06.06.2003 JP 2003162079
06.06.2003 JP 2003162080
06.06.2003 JP 2003162081
06.06.2003 JP 2003162082

(71) Applicant: MITSUBISHI CHEMICAL
CORPORATION
Tokyo 108-0014 (JP)

(72) Inventors:
• SUGYO, Yasunari,
MITSUBISHI CHEMICAL CORPORATION
Yokkaichi-shi, Mie 5108530 (JP)

• ITOH, Kiichi,
MITSUBISHI CHEMICAL CORPORATION
Yokkaichi-shi, Mie 5108530 (JP)
• HIMORI, Shunichi,
MITSUBISHI CHEMICAL CORPORATION
Yokkaichi-shi, Mie 5108530 (JP)
• MORI, Yoshiaki,
MITSUBISHI CHEMICAL CORPORATION
Yokkaichi-shi, Mie 5108530 (JP)
• ISHII, Taisuke,
MITSUBISHI CHEMICAL CORPORATION
Yokkaichi-shi, Mie 5108530 (JP)

(74) Representative: Zimmermann, Gerd Heinrich
Zimmermann & Partner
Postfach 33 09 20
80069 München (DE)

(54) WATER-ABSORBENT ARTICLES AND PROCESS FOR THE PRODUCTION THEREOF

(57) An absorbent article containing a water-absorbent resin composite comprising one nearly spherical water-absorbent resin particle and two or more fibers, wherein one or more of the fibers are partially embedded in the resin particle and are partially exposed from the resin particle, and wherein one or more of the fibers are never embedded in the resin particle but partially adhere to the surface of the resin particle. In the absorbent article, the water-absorbent resin particle is uniformly fixed on the fiber before, during and after water absorption, so that the water-absorbent resin can exist at a higher ratio to the fiber. Thus, the absorbent article characteristically has great softness and high shape stability.

Fig. 1

**Description**

FIELD OF THE INVENTION

The present invention relates to a water-absorbent article and a method for producing the same. The absorbent article in accordance with the invention can preferably be used in sanitary materials such as paper diapers and sanitary napkins, industrial materials needed for the absorption and retention of liquid wastes and the like, and agricultural materials such as freshness keepers of vegetables and the like, and water retentive agents.

DESCRIPTION OF THE BACKGROUND

Because most of commercially available water-absorbent resins are in powder, the water-absorbent resins are essentially dispersed uniformly on base materials such as tissue, non-woven fabric and cotton, so as to use the water-absorbent resins as sanitary materials such as sanitary napkins and paper diapers. However, it is difficult to stably fix the water-absorbent resins thus dispersed by such a method on the base materials. After dispersion, some of the water-absorbent resins are frequently aggregated locally. Additionally, the water-absorbent gel after water absorption is never fixed stably on the base materials but readily transfer from the base materials. In such a manner, there are formed regions in the absence of the water-absorbent gel, disadvantageously, to occasionally cause the leakage of body fluids when the body fluids re-infiltrate.

As a method for overcoming the problems, a method is known, including fixing water-absorbent resin powders on base materials with binders. Because the binders adhere on the surface of water-absorbent resins, however, the water absorption and swelling of the water-absorbent resin powders are inhibited, disadvantageously. Thus, such water-absorbent resins cannot exert their absorption potencies sufficiently. Additionally, an absorbent material with a fiber adhering on the surface of a water-absorbent resin is also proposed (JP-A-51-35685; JP-A-56-65630; and JP-A-58-163438). Although the water-absorbent resin is strongly bonded to the fiber before water absorption so the fiber is stably fixed well on the water-absorbent resin, such absorbent material is disadvantageous in that the fiber is readily dissociated out of the water-absorbent gel when the absorbent material absorbs liquid.

So as to solve the problems, an absorbent material is proposed, where a fiber is embedded inside a water-absorbent resin (JP-A-61-62463; JP-A-63-63723; JP-A-1-135350; and JP-B-8-19609). These absorbent materials can be produced by swelling a water-absorbent resin with water or the like, mixing or kneading a fiber into the water-absorbent resin to embed the fiber in the water-absorbent resin, and drying and subsequently grinding the resulting water-absorbent resin. The resulting absorbent materials have sharp and smooth surface and are partially angular. When the absorbent materials are pressurized to prepare the absorbent materials to a high density so as to cope with the design tendency of sanitary napkins and the like into thinner types, the water-absorbent resin therein collides with each other so that the angle is chipped off. Occasionally, the water-absorbent resin itself is distorted and cracked. Consequently, the water-absorbent resin in a free form after the water-absorbent resin is dissociated out of the fiber disadvantageously leaks out of the absorbent articles. Therefore, it has been difficult to allow an absorbent material with a water-absorbent resin stably fixed therein to have a higher density. During the kneading and mixing of a fiber into the water-absorbent gel, some of short fibers are completely embedded in the water-absorbent resin, so that the completely embedded fibers cannot exert their essential effect but trigger the inhibition of swelling. Because the fiber is not strongly bonded to the water-absorbent resin via polymerization, a void is generated between the fiber and the water-absorbent gel during swelling, so that the water-absorbent gel more readily transfers on the fiber. Via the kneading of the water-absorbent gel under mechanical pressure, the polymer chain therein is broken. Thus, the essential water absorption potency cannot be obtained. Additionally because the fiber never exists on the surface of the water-absorbent resin according to the production method, the fiber cannot instantly retain liquid, leading to poor liquid diffusion.

So as to solve the problems, an absorbent material is proposed, where a nearly spherical water-absorbent resin is discontinuously fixed on the surface of a non-fabricated fiber (JP-A-11-93073). These problems are overcome by the absorbent material. Since the non-fabricated fiber is bonded to each other through the water-absorbent resin to form a three-dimensional network, however, a restoring force from the fiber binding the water-absorbent resin together is eventually loaded when the absorbent material is pressurized. Even when a sanitary material in a thinner shape is produced from a highly densified absorbent material, the restoring force disadvantageously breaks the package or makes the thickness of the package larger when an individual user opens the package, so that the resulting shape is of a far larger thickness than the intended thickness, disadvantageously leading to the deterioration of the wear touch. Because the fiber simply adheres to the water-absorbent resin, the fiber is dropped out of the gel during swelling, so that the water-absorbent gel transfers, disadvantageously causing liquid leakage from the absorbent material.

It is a first object of the invention to provide an absorbent article with a water-absorbent resin particle uniformly fixed on a fiber prior to, during and after water absorption so the water-absorbent resin can exist on the fiber at a high ratio, and with great softness irrespective of the ultra-thin thickness, which can retain the shape stability for a long period of time. It is a second object of the invention to provide an absorbent article with hardly increase of the thickness thereof via the restoring force even after pressurization so as to make a thinner type. It is a third object of the invention to provide an absorbent article with a water-absorbent resin particle uniformly fixed on a fiber prior to, during and after water absorption so that the absorbent article has high shape stability for a long period of time. It is a fourth object of the invention to provide an absorbent article with appropriate flexibility and great wear touch. The first to fourth objects of the invention include providing sanitary materials, industrial materials and agricultural materials with such an absorbent article. Further, it is a fifth object of the invention to provide a method for producing an absorbent article with a water-absorbent resin particle uniformly fixed on a fiber prior to, during and after water absorption so that the water-absorbent resin can exist on the fiber at a high ratio, and with great softness irrespective of the ultra-thin thickness, which can retain the shape stability for a long period of time.

DISCLOSURE OF THE INVENTION

The first object of the invention is achieved by an absorbent article containing a water-absorbent resin composite, where the water-absorbent composite comprises one water-absorbent resin particle and two or more fibers, and where one or more of the two or more fibers are partially embedded inside the resin particle and are partially exposed from the resin particle and where one or more of the two or more fibers are never embedded inside the resin particle but partially adhere to the surface of the resin particle (referred to as "the first invention" hereinbelow).

The second object of the invention is achieved by an absorbent article containing a water-absorbent resin particle and a fiber and comprising a water-absorbent resin composite composition, where the restoring ratio represented by the following formula (1) is 50% or less (referred to as "the second invention" hereinbelow).

$$\text{restoring ratio (\%)} = (A-B)/B \times 100 \qquad (1)$$

In the formula, A represents the thickness of the water-absorbent resin composite composition after compressing the water-absorbent resin composite composition at a pressure of 100 Kg/cm$^2$ for 10 minutes and then storing the resulting water-absorbent resin composite composition at atmospheric pressure, a temperature of 25°C, and a humidity of 50% for 30 days; and B represents the thickness of the water-absorbent resin composite composition immediately after compressing the water-absorbent resin composite composition at a pressure of 100 Kg/cm$^2$ for 10 minutes.

The third object of the invention is achieved by an absorbent article containing a water-absorbent resin particle and a fiber, where the gel dropout ratio represented by the following formula (2) is 10% or less (referred to as "the third invention" hereinafter).

$$\text{Gel dropout ratio (\%)} = A/B \times 100 \qquad (2)$$

In the formula, A represents the weight of a dropped out water-absorbent gel after mounting a load of 3 Kg on the absorbent article after water absorption and shaking the absorbent article with an amplitude of 50 cm and the number of vibration being 80 vibrations/minute for 30 minutes; and B represents the weight of the water-absorbent gel before shaking.

The fourth object of the invention is achieved by an absorbent article containing a water-absorbent resin particle and a fiber and comprising a water-absorbent resin composite composition with a pliability of 5.0 to 9.5 cm as measured according to the heart loop method defined in JIS L-1096 (referred to as "the fourth invention" hereinbelow).

The fifth object of the invention is achieved by a method for producing an absorbent article including pressurizing a water-absorbent resin composite composition comprising the water-absorbent resin composite according to the first invention (referred to as "the fifth invention" hereinbelow) .

BRIEF DESCRIPTION OF THE DRAWINGS

Fig. 1 is a cross-sectional view depicting the constitution of the absorbent article.
Fig. 2 is a schematic view depicting a nozzle used for preparation of the water-absorbent resin composite.
Fig. 3 is a scanning electron microscope (SEM) photograph of the water-absorbent resin composite obtained in Production Example 1.
Fig. 4 is a schematic view of a pulp mixer.

Fig. 5 is a cross-sectional view describing an apparatus for measuring thickness.
Fig. 6 is a schematic view describing an apparatus for the measurement according to the heart loop method.
Fig. 7 is a cross-sectional view describing an apparatus for liquid absorption into the absorbent article.
Fig. 8 is a schematic view depicting the ro-tap shaker.
Fig. 9 is a view of the cutting line of a sample during the measurement of gel dropout ratio.

DETAILED DESCRIPTION OF THE INVENTION

The absorbent article and the method for producing the same in accordance with the invention are described below in more detail with reference to some preferred embodiments thereof. In the present description, the numerical range expressed by the wording "a number to another number" means the range between the former number indicating the lower limit of the range and the latter number indicating the upper limit thereof, inclusive of these limits.

First invention and fifth invention

The absorbent article in accordance with the first invention comprises a water-absorbent resin composite with a specific structure comprising a fiber and a water-absorbent resin as the essential constitutive elements, where a water-absorbent resin composite composition containing the composite is used as the absorption nucleus. The water-absorbent resin composite, the water-absorbent resin composite composition and the absorbent article in accordance with the first invention are described below in this order. The method for producing the absorbent article in accordance with the fifth invention is also described therein.

[Water-absorbent resin composite]

(Structure of the water-absorbent resin composite and roles of the constitutive elements)

The water-absorbent resin composite contained in the absorbent article in accordance with the invention comprises one nearly spherical, water-absorbent resin particle and two or more fibers. One or more fibers contained in the water-absorbent resin composite are partially embedded inside the water-absorbent resin particle and are partially exposed from the water-absorbent resin particle. Additionally, one or more fibers contained in the water-absorbent resin composite are never embedded inside the water-absorbent resin particle but partially adhere to the surface of the water-absorbent resin particle (referred to as "the water-absorbent resin composite of the invention" hereinbelow). In other words, the essential constitutive elements of the water-absorbent resin composite are the following three types: the water-absorbent resin particle, the fiber partially embedded in the water-absorbent resin particle, and the fiber adhering to the surface of the water-absorbent resin particle but never being embedded inside the water-absorbent resin particle. In the water-absorbent resin composite of the invention, the dry weight ratio of the fiber and the water-absorbent resin particle is preferably in the range of 1:1 to 1:1,000,000, more preferably 1:2 to 1:100,000, still more preferably 1:3 to 1: 10,000.

(Water-absorbent resin and roles thereof)

In the water-absorbent resin composite of the invention, the water-absorbent resin plays a role of absorbing fluids such as water, urine and blood, depending on the purpose of using the absorbent article of the invention.
The water-absorbent resin for use in the water-absorbent resin composite is a nearly spherical particle. The phrase "nearly spherical" herein referred to means a shape truly spherical or oval as a whole, and it may have microscopic protrusions and recesses (namely, wrinkles, protrusions, recesses, and the like) on the surface. It may also have voids such as small holes or cracks on the surface or inside. When the water-absorbent resin is in an irregular shape with sharp cut edges like other ground water-absorbent resins in the related art, disadvantageously, the water-absorbent resin irritates skin greatly and generates fine particles when the sharp cut edges are broken under a machinery load. The nearly spherical, water-absorbent resin particle to be used in accordance with the invention never has such drawbacks as described above. Compared with the water-absorbent resins in irregular shapes, additionally, the water-absorbent resin in accordance with the invention is advantageous in that the water-absorbent resin can be prepared into closest packing and therefore can be prepared into a higher density.

(Fiber partially embedded inside water-absorbent resin and roles thereof)

The fiber partially embedded in the water-absorbent resin plays a role of securing the fixability of the water-absorbent resin. The fiber also improves the fixability of the water-absorbent resin before and after water absorption.

Specifically, the fiber extending from the surface of the water-absorbent resin prevents the rotation and translation motions of the water-absorbent resin at extrusion pressure. Because the fiber is partially embedded in the water-absorbent resin and is never dissociated from the water-absorbent resin even after water absorption, the fiber can play an important role for the fixability of the water-absorbent resin after water absorption. The shape of the fiber to be used may satisfactorily be of a hollow shape or a side-by-side shape so as to elevate the water transferability.

When the fiber partially embedded in the water-absorbent resin is composed of a hydrophilic fiber, the fiber functions to improve the water transferability into the water-absorbent resin. Specifically, water can be led directly into the inside of the water-absorbent resin through the fiber. For allowing the fiber to more effectively exhibit this function, desirably, a fiber of high water transferability as described hereinbelow is selected and used.

Further, the fiber partially embedded in the water-absorbent resin functions to keep the independency of each water-absorbent resin composite. In the course of the polymerization of the precursor composite, the fiber prevents the water-absorbent resins from fusing with each other via the steric hindrance of each fiber. In other words, the fiber extending from the surface of the water-absorbent resin blocks the water-absorbent resins not to contact with each other in the progress of polymerization. In such a manner, the fiber blocks the water-absorbent resins not to fuse with each other. Consequently, each water-absorbent resin composite (precursor) keeps its independency and prevents its deposition onto the wall of a reactor during the production step or treatment step and additionally can retain the opening property.

Meanwhile, the fiber partially embedded in the water-absorbent resin allows the water-absorbent resin composite to be appropriately entangled each other physically. When plural such composites are assembled into a mass, the fiber therein further gives such shape retentivity to the composite mass under a load of about the weight thereof that the mass is never fallen into pieces. In other words, the mass comprising the water-absorbent resin composite can retain the shape retentivity by itself, even without any addition of free fiber or the like. Therefore, the water-absorbent resin composite has an extremely great feature such that the water-absorbent resin composite can form a mass with an opening property and the shape retentivity.

Because the fiber composing the water-absorbent resin composite gives a soft touch and because the water-absorbent resin contained in the composite is nearly spherical, furthermore, the composite is very soft even at its dry state at extrusion pressure. Therefore, the composite is preferable for sanitary materials and the like.

(Fiber adhering to the surface of water-absorbent resin but never being embedded therein and roles thereof)

The fiber adhering to the surface of the water-absorbent resin but never being embedded in the water-absorbent resin is effective for securely retaining the fixability of the water-absorbent resin before water absorption. After the water-absorbent resin is swollen, the fiber on the surface of the water-absorbent resin functions to form voids among the individual water-absorbent resins to securely retain the water channel. So as to allow the function to be exerted, the fiber does not necessarily adhere to the water-adsorbent resin after water absorption. However, preferably, the fiber is at least arranged closely on the surface of the water-absorbent resin. In accordance with the invention, thus, the fiber advantageously adheres to the surface of the water-absorbent resin before water absorption. For the formation of voids among the individual water-absorbent resins to securely retain the water channel, a fiber with certain rigidity is sometimes preferably used. Such a fiber can effectively secure the fixability of the water-absorbent resin before water absorption, together with the fiber embedded in the water-absorbent resin. The fiber used may be of a hollow shape or a side-by-side shape so as to enhance the diffusibility.

In the case that the fiber adhering to the surface of the water-absorbent resin but never being embedded in the water-absorbent resin is composed of a hydrophilic fiber, the fiber is effective for preventing the blocking (lumps-forming) phenomenon that the water-absorbent resin is put in contact with each other via the swelling of the water-absorbent resin during water absorption to block the water channel. In other words, the fiber functions to evenly transfer and diffuse water during water absorption. In the case that the fiber adhering to the surface of the water-absorbent resin but never being embedded in the water-absorbent resin is composed of a hydrophobic fiber, alternatively, the fiber exerts an action to improve water diffusibility among the individual water-absorbent resins. The fiber adhering to the surface of the water-absorbent resin but never being embedded in the water-absorbent resin permits the retention of the independency of each water-absorbent resin composite, via the same action of the aforementioned "fiber embedded in the water-absorbent resin", to effectively form a mass with very soft touch, having an opening property and the shape retentivity.

(Preferable ranges of particle size and fiber length)

The average particle size of the water-absorbent resin particle for use in accordance with the invention is preferably 50 to 1,000 $\mu$m. The average particle size of the water-absorbent resin particle is more preferably 100 to 900 $\mu$m. The average particle size of the water-absorbent resin particle is particularly preferably 200 to 800 $\mu$m. As described

below, the fiber preferable for use in accordance with the invention is of a fiber length of 50 to 50,000 $\mu$m. More preferably, the fiber length is 100 to 30,000 $\mu$m. Still more preferably, the fiber length is 500 to 10,000 $\mu$m. So as to obtain the shape in accordance with the invention, the ratio of the particle size: the fiber length is preferably 2:1 to 1:1,000. The ratio is more preferably 1:1 to 1:500, particularly preferably 1:2 to 1:100.

(Composite effect of individual fibers)

In general, securing the fixability of the water-absorbent resin is never compatible with securing the water absorption potencies such as retentivity and water absorption potency under pressure. In order to secure sufficient fixability not only before water absorption but also after water absorption, the adhesion force between the water-absorbent resins which is far exceeding the expansion force due to water absorption is needed even after water absorption. This specifically causes the inhibition of the water absorption and swelling of the water-absorbent resin of itself, resulting in poor water absorption potency. When the adhering face is allowed to freely swell so as to securely retain the water absorption potencies such as retentivity and water absorption potency under pressure, meanwhile, the adhering face is damaged concurrently, so that sufficient fixability cannot be imparted.

For the water-absorbent resin composite of the invention, the fiber partially embedded in the water-absorbent resin particle and partially exposed from the water-absorbent resin particle and the fiber partially adhering to the surface of the water-absorbent resin particle but being never embedded in the water-absorbent resin particle are both essential. The water-absorbent resin composite containing the former fiber alone cannot sufficiently effectively prevent the blocking (lumps-forming) phenomenon during water absorption. In the water-absorbent resin composite containing the latter fiber alone, alternatively, the fixability of the water-absorbent resin after water absorption is insufficient. Accordingly, both the fibers are essential for the exertion of the above-mentioned effects prior to, during and after water absorption. The coexistence of both the fibers allows the compatibility of securing the fixability of the water-absorbent resin and securing the water absorption potencies, which are essentially not compatible to each other. Specifically, the water-absorbent resin composite has a prominent feature that the water-absorbent resin composite can securely have not only retentivity but also water absorption potencies under pressure while the water-absorbent resin composite can securely have sufficient fixability not only before water absorption but also after water absorption.

The types of the two fibers may be the same or different, and may appropriately be selected, depending on the purpose of the use and for the exertion of the individual effects thereof.

(Opening property)

One of the features of the water-absorbent resin composite is not only that the assembly of the water-absorbent resin composite has an opening property but also that the water-absorbent resin composite composition comprising the composite can be allowed to have an opening property. Such features can be securely obtained by the fact that the individual composites are substantially independent of each other. Specifically, it is desirable that the fibers composing one such composite do not substantially adhere to any other composite. Depending on the production conditions, preferably, the length of the fibers to be used is appropriately selected. The opening property can be evaluated on the basis of the easiness in worsted spinning and on the damage status of the water-absorbent resin particle after worsted spinning, as mentioned in the following section about the method for the measurement.

(Shape retentivity)

The water-absorbent resin composite of the invention is characterized in that not only the assembly of the composite has the shape retentivity but also the water-absorbent resin composite composition comprising the composite can be allowed to have the shape retentivity. As mentioned hereinabove, the binding fiber in the water-absorbent resin composite allows the individual water-absorbent composites to be appropriately entangled with each other physically. Even when the water-absorbent resin composite composition is prepared into a mass, the mass is never readily broken into pieces at a load of the weight thereof. Such shape retentivity is given.

(Water-absorbent resin composite composition)

On a needed basis depending on the use and the like, a fiber is further added to the water-absorbent resin composite, to prepare the water-absorbent resin composite composition. In the composition, the fiber is a fiber never embedded in or never adhering to the water-absorbent resin. Via the addition of the fiber, the softness, the soft touch, the water transferability, the water passability, the water diffusibility, the gas permeability and the like can be further improved. Via appropriate selection of a fiber to be added, the opening property of the resulting composition per se can be secured.

The composition can generally be produced by appropriately mixing and dispersing a fiber into the water-absorbent resin composite produced. Otherwise, the composition may be obtained by feeding, mixing and dispersing such a fiber into the water-absorbent resin composite during the production of the water-absorbent resin composite, by a method substantially in no contact with the water-absorbent resin under polymerization or the water-absorbent resin in the water-absorbent resin composite.

(Water-absorbent resin)

The water-absorbent resin for use in accordance with the invention can be produced, using the following polymerizable monomer and an initiator.

Any polymerizable monomer may be used irrespective of the type thereof, as long as a water-absorbent resin can be produced from the monomer. A polymerizable monomer of which the polymerization is initiated by a redox initiator is particularly preferable for use herein. Generally, the polymerizable monomer is preferably water-soluble.

Typical and preferred examples of the monomer for use in accordance with the invention are aliphatic unsaturated carboxylic acids or salts thereof. Specifically, the monomer includes for example unsaturated monocarboxylic acids or their salts thereof, such as acrylic acid or salts thereof, methacrylic acid or salts thereof; or unsaturated dicarboxylic acids or salts thereof, such as maleic acid or salts thereof, itaconic acid or salts thereof. These may be used either singly or in combination of two or more thereof. Among them, acrylic acid or salts thereof, and methacrylic acid or salts thereof are preferable. Acrylic acid or salts thereof are particularly preferable....

As mentioned hereinabove, the polymerizable monomer from which the water-absorbent resin for use in accordance with the invention is produced is preferably an aliphatic unsaturated carboxylic acid or salts thereof. As the aqueous solution of the polymerizable monomer, therefore, preference is given to an aqueous solution containing an aliphatic unsaturated carboxylic acid or a salt thereof as the main ingredient. By the phrase "containing an aliphatic unsaturated carboxylic acid or a salt thereof as the main ingredient" is meant that the content of the aliphatic unsaturated carboxylic acid or the salt thereof is 50 mol% or more, preferably 80 mol% or more of the total amount of the polymerizable monomers therein.

The salts of aliphatic unsaturated carboxylic acid are generally water-soluble salts, including, for example, alkali metal salts, alkaline earth metal salts and ammonium salts. The degree of the neutralization of the salts may be suitably determined on the basis of the purpose thereof. For salts of acrylic acid, desirably, 20 to 90 mol% of the carboxyl group therein is neutralized into an alkali metal salt or an ammonium salt. If the degree of the partial neutralization of the acrylic acid monomer is less than 20 mol%, the water absorption potency of the resulting water-absorbent resin may tend to be significantly deteriorated.

For neutralizing the acrylic acid monomer, alkali metal hydroxides and bicarbonates, ammonium hydroxide and the like may be used. Alkali metal hydroxides are preferable. Specific examples thereof include sodium hydroxide and potassium hydroxide.

Other than the aliphatic unsaturated carboxylic acids described above, in accordance with the invention, polymerizable monomers copolymerizable with the above-mentioned aliphatic unsaturated carboxylic acids may be copolymerized satisfactorily within a range of an amount thereof without any deterioration of the generated water-absorbent resin. Examples of such polymerizable monomers include (meth)acrylamide, (poly)ethylene glycol (meth)acrylate and 2-hydroxyethyl (meth)acrylate and additionally include alkyl acrylates such as methyl acrylate or ethyl acrylate although they are poorly water-soluble monomers. In this description, the term "(meth)acryl" means both "acryl" and "methacryl".

The polymerizable monomers from which the water-absorbent resin is generated among those polymerizable monomers described above may also be used as main monomers in the "aqueous solution of a polymerizable monomer from which a water-absorbent resin can be generated", not as the auxiliary components for the aliphatic unsaturated carboxylic acid or salts thereof.

The aliphatic unsaturated carboxylic acid or salts thereof, especially acrylic acid or a salt thereof may form self-crosslinked polymers by themselves, but may be combined with a crosslinking agent to actively form a crosslinking structure. When a crosslinking agent is used in combination, generally, the water absorption performance of the resulting water-absorbent resin is enhanced. As the crosslinking agent, polyvinyl compounds copolymerizable with the above-mentioned polymerizable monomers are preferably used and include for example N,N'-methylene-bis(meth)acrylamide and (poly)ethylene glycol di(meth)acrylates, as well as water-soluble compounds having at least two functional groups capable of reacting with carboxylic acids, for example, polyglycidyl ethers such as ethylene glycol diglycidyl ether and polyethylene glycol diglycidyl ether. Among them, N,N'-methylene-bis(meth)acrylamide is particularly preferable. The amount of the crosslinking agent to be used may be 0.001 to 1% by weight (sometimes abbreviated as "wt%" hereinafter), but preferably 0.01 to 0.5% by weight of the amount of the monomer charged for polymerization.

In the aqueous polymerizable monomer solution containing the above-mentioned aliphatic unsaturated carboxylic acid or a salt thereof as the main ingredient, the concentration of the polymerizable monomer may be preferably 20 wt% or more, more preferably 25 wt% or more. When the monomer concentration is lower than 20 wt%, the water absorption potencies of the water-absorbent resins obtained after polymerization are likely unsatisfactory. The upper limit of the polymer concentration may be about 80 wt% in view of the processability of the polymerization solution.

The polymerization initiator for use in accordance with the invention may be any of those usable in radical polymerization in aqueous solution. Such initiator includes inorganic and organic peroxides, for example persulfate salts of ammonium and alkali metals, especially potassium, hydrogen peroxide, t-butyl peroxide, acetyl peroxide, etc.

Initiators known as azo initiators may also be used. For example, 2,2'-azobis(2-amidinopropane) dihydrochloride water-soluble in some degree is included.

The polymerization is initiated through decomposition of the radical polymerization initiator. Pyrolysis is one well-known process. A polymerization initiator not heated may often be added to a monomer reaction solution that has been previously heated up to the decomposition point of the polymerization initiator to initiate the polymerization. This case is also within the technical scope of pyrolysis.

The initiator preferably used in accordance with the invention is a combination of an oxidizing agent and a reducing agent composing a redox system water-soluble in some degree.

The oxidizing agent includes, for example, hydrogen peroxide, persulfates such as ammonium persulfate, potassium persulfate; as well as t-butyl hydroperoxide, cumene hydroperoxide, ceric salts, permanganates, chlorites, hypochlorites, etc. Among them, especially preferred is hydrogen peroxide. The amount of the oxidizing agent to be used may be 0.01 to 10 wt% and preferably 0.1 to 2 wt% of the polymerizable monomer.

The reducing agent may form a redox system with the oxidizing agent and specifically includes for example sulfite salts such as sodium sulfite and sodium hydrogen sulfite, sodium thiosulfate, cobalt acetate, copper sulfate, ferrous sulfate, L-ascorbic acid or alkali metal salts of L-ascorbic acid. Among them, L-ascorbic acid or alkali metal salts of L-ascorbic acid are particularly preferable. These reducing agents may be used at 0. 001 to 10 wt %, preferably 0.01 to 2 wt % of the polymerizable monomer.

(Fiber)

As the fiber, for example, synthetic fibers, natural fibers, semi-synthetic fibers, and inorganic fibers may be used.

A fiber is selected and used, on the basis of the purpose of using the water-absorbent resin composite, and the role of each fiber composing the water-absorbent resin composite. When the composite is used in a water-absorbent article, for example, highly hydrophilic fibers such as pulp, rayon, cotton, regenerated cellulose and other cellulose-series fibers are preferably selected. From the standpoint of water transferability, highly hydrophilic fibers are preferable. For the use in sanitary materials, in particular, pulp is preferable, additionally from the standpoint of great touch. Any pulp type is usable with no specific limitation. However, the pulp includes for example mechanical pulps such as tree-ground pulp; chemical and mechanical pulps such as semi-chemical pulp and chemical ground pulp; chemical pulps such as sulfite pulp, sulfate pulp, soda pulp, pulp according to the nitrate method, and pulp according to the chlorine method; and regenerated pulps such as mechanically ground or pulverized paper from paper once made, or regenerated paper from waste paper as mechanically ground or pulverized waste paper.

Taking account of environmental pollution, additionally, biodegradable synthetic fibers such as polylactate fiber and aliphatic polyester may satisfactorily be used in place of non-biodegradable synthetic fiber.

From the standpoint of the fixability of the water-absorbent resin, preferably, each fiber strongly adheres to the water-absorbent resin before water absorption and after water absorption. It is known that the adhesion of a highly hydrophilic fiber to another highly hydrophilic fiber is generally high. In accordance with the invention, a fiber with high affinity to the water-absorbent resin is preferably used. Generally, a fiber with high affinity to generally hydrophilic water-absorbent resins is a hydrophilic fiber. The affinity between a hydrophilic water-absorbent resin and a fiber to be used can be assayed as the contact angle of water on the surface of a fiber material as a quantitative indicator. A smaller contact angle of water on the surface of a fiber material indicates higher affinity between the water-absorbent resin and the fiber, also indicating higher adhesion. In contrast, a larger contact angle indicates lower affinity, indicating lower adhesion. In such sense, the contact angle of water on the surface of a fiber material is preferably 60° or less, more preferably 50° or less, most preferably 40° or less. The contact angle varies depending on the shape of a fiber material to be measured, and the surface smoothness thereof. In accordance with the invention, the contact angle means the contact angle of a fiber material placed on smooth surface such as film and sheet with distilled water. The contact angle can be measured, using an apparatus described below.

The hydrophobic fiber may satisfactorily be used after the surface of the hydrophobic fiber is made into hydrophilicity, so as to make the contact angle of water on the surface of the fiber material to 60° or less. Therefore, the surface thereof may be modified with one or more known anion-series, cation-series and nonion-series surfactants. For example, the surface thereof may be made into hydrophilicity, by a method including spraying and coating directly such surfactants on hydrophobic fibers, a method including coating the surfactants during or after the formation of such fibers or non-woven fabrics, a method including adding the surfactants to a polymer composition before spinning the composition into such fibers.

From the standpoints of water passability and water diffusibility, the hydrophobic fiber may be used in combination with a hydrophilic fiber. For example, polyester-series, polyethylene-series, polypropylene-series, polystyrene-series, polyamide-series, polyvinyl alcohol-series, polyvinyl chloride-series, polyvinyl vinylidene-series, polyacrylonitrile-series, polyurea-series, polyurethane-series, polyfluoroethylene-series and polyvinylidene cyanide-series fibers may be used. For example, a hydrophilic fiber may be selected as a fiber partially embedded in the water-absorbent resin particle and partially exposed from the water-absorbent resin particle, while a hydrophobic fiber may be selected as a fiber never embedded in the water-absorbent resin particle but partially adhering to the surface of the water-absorbent resin particle. When such an embodiment is selected, such a hydrophobic fiber exerts its function to improve the water diffusibility in the water-absorbent resins.

Further, two or more fiber types may be used in combination. Then, the fiber types and mix ratio thereof may be selected within a range with no inhibition of water passability, although the selection depends on the use of the water-absorbent resin composite composition. Additionally, the mix ratio of the fibers in the water-absorbent resin composite composition may be gradually increased or decreased or the individual fibers therein may be locally positioned for use.

From the standpoint of blocking inhibition, importantly, the fibers should be selected in view of the fiber rigidity and fiber size, as described below.

The fiber preferable for use in accordance with the invention is of an average fiber length of 50 to 50, 000 $\mu$m. More preferably, the average fiber length is 100 to 30,000 $\mu$m. Still more preferably, the average fiber length is 500 to 10,000 $\mu$m. When the fiber length is longer than 50, 000 $\mu$m, the fiber adheres to plural such water-absorbent resins, so that the individual water-absorbent resin composites cannot be retained independently. Therefore, the opening of the composition containing the composite is likely to be difficult. In contrast, a shorter fiber length than 50 $\mu$m likely causes difficulty in the embedding of the fiber in the water-absorbent resin and the adhesion thereof to the water-absorbent resin.

So as to allow the water-absorbent resin composite to have a desired shape, the ratio of the water-absorbent resin particle size: the fiber length is preferably 2:1 to 1:1,000. More preferably, the ratio is 1:1 to 1: 500. Particularly preferably, the ratio is 1:2 to 1:100.

In accordance with the invention, a fiber with a fiber size of 0.1 to 500 dtex is preferable. A fiber with a fiber size of 0.1 to 100 dtex is more preferable. A fiber with a fiber size of 1 to 50 dtex is still more preferable. A fiber with a fiber size of 1 to 10 dtex is particularly preferable. When the fiber size is more than 500 dtex, the fiber has such an excessively large rigidity to cause difficulty in the embedding in the water-absorbent resin or the adhesion to the water-absorbent resin. Additionally, the compact molding thereof is difficult in that case, unpreferably for producing thinner article types. For uses such as sanitary napkins and the like, such a fiber brings about unfavorable touches such as very stiff and itchy touch. When the fiber size is less than 0.1 dtex, the fiber is too thin to securely procure the water transferability and diffusibility in some case. Because the rigidity is also poor, lumps-formation cannot be prevented.

Additionally, nonlinear fibers such as curled fibers and branched fibers may also be used as fiber-like materials.

Instead of non-biodegradable synthetic fibers, biodegradable synthetic fibers such as polylactate fibers and aliphatic polyester may be used in light of environmental pollution.

From the various standpoints described above, the fiber type, the fiber length, the fiber size, and the fiber shape may appropriately be selected.

The fiber is preferably dispersed as uniformly as possible in a microscopic fashion. Generally, fibers are likely entangled together to form a fiber mass. The apparent fiber mass size is preferably 20 mm or less, more preferably 10 mm or less, and most preferably 5 mm or less. It is needless to say that the fiber is preferably independent of each other. So as to securely achieve uniformity, generally, an opening approach is used. Herein, the term "opening" includes both the concepts of splitting and fibrillation. Splitting includes splitting sheet-like materials such as nylon into strips and fibers. Fibrillation includes cutting up and tearing a raw material cellulose into pulp, and the like.

For specific procedures therefor, there may be used cotton-spinning type, worsted-spinning type, woolen-spinning type, linen-spinning type, silk-spinning type or rotor blade type grinding machines, hammer type grinding machines, pulp fibrillating machines and the like, as introduced in *Fiber Handbook (processing edition)* (edited by the Society of Fiber Technology of Japan, published by Maruzen, 1969), page 18 and thereafter. Additionally, a process known as flocking process may also be used, the process including electrically charging fibers to make the fibers substantially independent of each other for uniform dispersion thereof, utilizing the electrostatic repulsion of the fibers.

[Water-absorbent resin composite composition]

(Constitution)

The water-absorbent resin composite composition of the invention characteristically comprises the water-absorbent resin composite. For example, a water-absorbent resin composite composition may be produced by mixing a fiber with the water-absorbent resin composite in deposition or the water-absorbent resin composite opened and independently existing, so that the water-absorbent resin composite composition in mixture of the water-absorbent resin composite and the fiber at an appropriate ratio can be produced. With no specific limitation, any fiber may be mixed in the water-absorbent resin composite. For example, the water-absorbent resin composite composition of the invention may contain one or more fibers never embedded in or never adhering to the water-absorbent resin.

Then, the dry weight ratio of "the fiber never embedded in or adhering to the water-absorbent resin" and the water-absorbent resin is preferably 90:10 to 5:95, more preferably 90:10 to 35:75, and most preferably 85:15 to 35:65. When the dry weight ratio of "the fiber never embedded in or adhering to the water-absorbent resin" and the water-absorbent resin exceeds 90:10, substantially, the effect of the water-absorbent resin is hardly exerted, with the resultant smaller bulk density. When the dry weight ratio of "the fiber never embedded in or adhering to the water-absorbent resin" and the water-absorbent resin is lower than 5: 95, occasionally, the softness, the soft touch, the water transferability, the water passability, the water diffusibility, the gas permeability and the like cannot be further improved sufficiently.

The water-absorbent resin composite composition in accordance with the invention may satisfactorily contain a composite comprising a fiber and a water-absorbent resin, other than the water-absorbent resin composite of the invention. For example, the water-absorbent resin composite composition of the invention may satisfactorily comprise a water-absorbent resin composite comprising one or more water-absorbent resin particles and one or more fibers, where the water-absorbent resin particles are approximately spherical and the one or more fibers are partially embedded in the water-absorbent resin particles and are partially exposed from the water-absorbent resin particles and where none of the fibers adheres to the surface of the resin particles. Additionally, the water-absorbent resin composite composition of the invention may satisfactorily comprise a water-absorbent resin composite comprising one or more water-absorbent resin particles and one or more fibers, where the water-absorbent resin particles are approximately spherical and the one or more fibers partially adhere to the surface of the water-absorbent resin particles and where none of the fibers is embedded in the resin particles. The water-absorbent resin composite composition in accordance with the invention may contain the water-absorbent resin composite at preferably 0.1 or more, more preferably 0. 2 or more and most preferably 0.3 or more on a weight fraction ratio basis. The bulk density of the water-absorbent resin composite composition in accordance with the invention is preferably 0.20 to 1.10 g/cm$^3$, more preferably 0.30 to 0.85 g/cm$^3$, and still more preferably 0.40 to 0.85 g/cm$^3$.

(Fiber never embedded in or never adhering to the water-absorbent resin)

As the fiber, for example, synthetic fibers, natural fibers, semi-synthetic fibers, and inorganic fibers may be used.

A fiber is selected and used, on the basis of the purpose of using the water-absorbent resin composite composition. When the composition is to be used in an absorbent article, for example, highly hydrophilic fibers such as pulp, rayon, cotton, regenerated cellulose and other cellulose-series fibers are preferably selected. In respect of water transferability, highly hydrophilic fibers are preferable. For the use in sanitary materials, in particular, pulp is preferable, additionally from the standpoint of great touch.

Hydrophobic fibers may also be used. For example, polyester-series, polyethylene-series, polypropylene-series, polystyrene-series, polyamide-series, polyvinyl alcohol-series, polyvinyl chloride-series, polyvinyl vinylidene-series, polyacrylonitrile-series, polyurea-series, polyurethane-series, polyfluoroethylene-series and polyvinylidene cyanide-series fibers may be used. These hydrophobic fibers when used can improve the water passability and water diffusibility in the resulting composition.

The affinity of a fiber to be used with the water-absorbent resin or the affinity thereof with the water-absorbent resin composite is not specifically limited.

The fiber type to be used may satisfactorily be the same as "the fiber embedded in or adhering to the water-absorbent resin". For example, a hydrophilic fiber may be selected as "the fiber embedded in or adhering to the water-absorbent resin", while a hydrophobic fiber may be selected as "the fiber never embedded in or adhering to the water-absorbent resin". When such an embodiment is selected, the hydrophobic fiber can exert the function to improve the water diffusibility in the water-absorbent resin composites.

The mix ratio of two or more fiber types when used in combination may preferably be selected within a range with no inhibition of the water transferability in the resulting absorbent article. Additionally, the mix ratio of the fibers may be gradually increased or decreased or the individual fibers may be locally positioned for use.

From the standpoint of blocking inhibition, importantly, the fibers should be selected in view of the fiber rigidity and fiber size, as described below.

The fiber preferable for use as the fiber never embedded in or adhering to the water-absorbent resin is of an average fiber length of 50 to 100, 000 μm. More preferably, the average fiber length is 100 to 50,000 μm. Still more preferably, the average fiber length is 500 to 20,000 μm. When the fiber length is longer than 100,000 μm, the resulting composition is sometimes hardly opened. In contrast, a shorter fiber length than 50 μm is problematic in that the fiber may be leaked from the resulting composition because the fiber per se is at a high transferability.

As the fiber never embedded in or adhering to the water-absorbent resin, a fiber with a fiber size of 0.1 to 500 dtex is preferable. A fiber with a fiber size of 0.1 to 100 dtex is more preferable. A fiber with a fiber size of 1 to 50 dtex is still more preferable. A fiber with a fiber size of 1 to 10 dtex is particularly preferable. When the fiber size is more than 500 dtex, the fiber has such large rigidity that the fiber is hardly mixed into the water-absorbent resin or be hardly compressed or molded. Such fiber is not preferable for producing a thinner article. For uses such as sanitary napkins and the like, such fiber causes unfavorable touches such as very stiff and itchy touch. When the fiber size is less than 0.1 dtex, the fiber is too thin to securely procure the water transferability and diffusibility. Because the rigidity is also insufficient, lumps-formation cannot be prevented.

From the various standpoints described above, the fiber type, the fiber length, and the fiber size may appropriately be selected.

(Production method)

The method for producing the water-absorbent resin composite in accordance with the invention is not specifically defined, so long as the water-absorbent resin composite satisfying the requirements claimed herein can be provided by the method.

A preferable method for producing the water-absorbent resin composite comprises adding a redox-type polymerization initiator to an aqueous solution of a polymerizable monomer from which the water-absorbent resin can be generated, for example, to an aqueous solution of a polymerizable monomer comprising an aliphatic unsaturated carboxylic acid or its salt as the main ingredient, to initiate the polymerization of the monomer, preparing the reaction mixture containing the polymerizable monomer and the generated polymer after the initiation of the polymerization under polymerization into liquid droplets in a gas phase, putting the resulting liquid droplets in contact with fibers fed into the gas phase to prepare a water-absorbent resin composite precursor, and finally completing the polymerization to recover the water-absorbent resin composite.

One preferred method of polymerizing the liquid droplets together in a gas phase comprises mixing a first solution comprising an aqueous solution of a polymerizable monomer containing either one of an oxidizing agent and a reducing agent composing the redox-type polymerization initiator, with a second solution comprising an aqueous solution containing the remaining agent of the redox-type polymerization initiator and optionally the polymerizable monomer if desired, in a gas phase, to initiate the polymerization of the monomer.

Specifically, the process includes individually ejecting the first solution and the second solution separately through different nozzles in such a manner that the two solutions flowing from the nozzles may colloid with each other at a cross angle of at least 15 degrees and in their states of liquid column. By colliding the two solutions each other at such a cross angle, a part of the flowing energy from the nozzles can be utilized for mixing the solutions. The cross angle at which the first and second solutions flowing from the individual nozzles is appropriately determined, depending on the properties of the polymerizable monomer used, the flow ratio of the solutions and the like. For example, the cross angle can be smaller at larger linear speeds of the solutions.

In this case, further, the temperature of the first solution may be generally from room temperature to about 60°C, preferably from room temperature to about 40°C; and the temperature of the second solution may also be generally from room temperature to about 60°C, preferably from room temperature to about 40°C.

In the manner described above, the individual aqueous solutions ejected from the nozzles are allowed to collide with each other in their states of liquid column, to combine the two solutions together. After the combination, the solutions are still in the form of liquid column. Their states are maintained for a certain period of time. Subsequently, the liquid columns are broken into liquid droplets. The polymerization of the liquid droplets thus formed progresses in a gas phase.

The size of the liquid droplets is about 5 to 3,000 $\mu$m in diameter. In order to progress the polymerization of the liquid droplets in contact with a fiber to form an appropriate water-absorbent resin composite, the size of the liquid droplets is particularly preferably within a range of 50 to 1,000 $\mu$m. The spatial density of the liquid droplets in a reactor is preferably 10 to 10,000 g/m$^3$. When the size exceeds the upper limit, some of the water-absorbent resins exist in no contact with the fiber. When the size is less than the lower limit, a part of the fiber exists in no contact with the water-absorbent resin. Thus, disadvantageously, the yield of the water-absorbent resin composite may be relatively decreased.

The gas in the gas phase providing a reaction field for the initiation of the polymerization and the formation of the liquid droplets in the course of the polymerization is preferably a gas inert to the polymerization and includes for example nitrogen, helium or carbon dioxide. The gas may also be air. The humidity of the gas, including the case where a gas simply consisting of water vapor alone, is not specifically defined. When the humidity is too low, however, the water in the aqueous solution of the polymerizable monomer evaporates before the monomer polymerization proceeds. As a result, the polymerizable monomer deposits. Consequently, the polymerization speed may be extremely lowered or the polymerization may be terminated in the course of reaction. The temperature of the gas may be from room temperature or more to 150°C or less, preferably 100°C or less. The gas flow direction may be any of counter flow or parallel flow, relative to the direction in which the liquid columns and the liquid droplets run. When the retention time of the liquid droplets in the gas phase is essentially to be prolonged, i.e. when the degree of polymerization ratio of the polymerizable monomer has to be increased as well as the viscosity of the liquid droplets has to be increased, the gas flow is preferably a counter flow (in the direction opposite to the gravity).

(Fiber feed port)

The conversion ratio of the polymerizable monomer in the liquid droplets during the contact of the liquid droplets with a fiber is preferably in the range of 0 to 90%. The ratio is more preferably 0 to 70%, most preferably 0 to 60%. At a conversion ratio of 90% or more, possibly, the fiber used can neither be embedded in nor adhere to the water-absorbent resin.

The water-absorbent resin composite with the structure described above in accordance with the invention may also be produced by feeding a fiber into a reaction field at a reaction stage with an equal conversion ratio of the polymerizable monomer. However, preferably, the water-absorbent resin composite of the invention may be produced by feeding a fiber into reaction fields at two or more stages with different conversion ratios of the polymerizable monomer. Therefore, the fiber is preferably fed from multiple feed ports. Specifically when the fiber is to be partially embedded in the water-absorbent resin, desirably, the fiber is put in contact at a stage with a relatively smaller conversion ratio of the polymerizable monomer. In the case that the fiber is not to be embedded in the water-absorbent resin but to adheres to the surface of the water-absorbent resin, desirably, the fiber is put in contact at a stage with a relatively larger conversion ratio of the polymerizable monomer.

For preparing both the fiber partially embedded in the water-absorbent resin and the fiber never embedded in the water-absorbent resin but adhering to the surface thereof, the difference in the conversion ratio of the polymerizable monomer between the contact fields of the individual fibers with the polymerizable monomer is preferably in the range of 10% to 80%, more preferably 10 to 70%, most preferably 10 to 60%. The conversion ratio at each contact field is appropriately determined, depending on the polymerizable monomer type and the fiber type.

(Fiber transfer method)

As a method for feeding a fiber to make the fiber in contact with the liquid droplets in the course of polymerization, any known transfer method may be used. The spatial density of the fiber in a reactor is preferably in the range of 0.005 to 1, 000 $g/m^3$. When the spatial density exceeds the range, then, some of the fibers exist in no embedding in the water-absorbent resin. When the spatial density is less than the lower limit, some of the water-absorbent resins exist without the embedded fiber therein, to relatively decrease the yield of the water-absorbent resin composite, disadvantageously. So as to feed a fiber at a state as finely as possible and as uniformly as possible, preferably, the fiber is fed in a mix phase flow with a gas. As the gas to be used herein, the gases providing the reaction field as listed above are preferably used. Among them, air is preferable from the economical standpoint and the standpoint of reducing environmental burdens.

The mix weight ratio of the fiber and the gas to be fed in the mix phase flow is 1:1 or less, while the linear speed of the gas is preferably within a range of 1 to 50 m/s. When the linear speed of the gas exceeds 50 m/s, the move of the reaction mixture in the way of polymerization at a reaction field is disordered disadvantageously, sometimes resulting in a deposition problem on the inner face of the reactor. When the linear speed is less than the lower limit, the uniformity of the fiber may not sometimes be maintained.

The temperature of the gas fed in the mix phase flow is desirably selected within a range without any significant inhibition of the polymerization. In that sense, the temperature is preferably from room temperature or more to 150°C or less, preferably 100°C or less. From the viewpoint of fiber transfer, the humidity in the gas is preferably lower. When the humidity is too low, however, the humidity in the reactor may be lowered, to evaporate water from the aqueous monomer solution to deposit the monomer before the monomer polymerization progresses. As a result, possibly, the polymerization speed is extremely lowered or the polymerization is terminated in the course of reaction.

(Opening of water-absorbent resin composite)

The water-absorbent resin composite is recovered as a deposit. Because the individual water-absorbent resin composites are independent of each other, the water-absorbent resin composite can be opened readily. For opening, the opening method described in the description of fiber is appropriately applicable in the same manner. An apparatus never capable of breaking the water-absorbent resin via the mechanical impact or the conditions therefor are preferable.

(Other additional steps)

Other additional steps may be added to the method of producing the water-absorbent resin composite of the invention. For example, the steps are a step of processing the remaining monomer; a surface-crosslinking step; and a step of adding some additives such as catalysts, reducing agents, deodorants, human urine stabilizers, and antimicrobial agents to the composite for imparting various additional functions thereto.

The method for processing the remaining monomer includes for example (1) a method of further polymerizing the monomer, (2) a method of converting the monomer into some other derivative, and (3) a method of removing the monomer.

The method (1) of further polymerizing the monomer includes a method of further heating the composite of the water-absorbent resin and the fiber; a method of adding a catalyst or a catalyst component capable of promoting the monomer polymerization to the water-absorbent resin composite and subsequently heating the resulting mixture; an ultraviolet irradiation method; and an electromagnetic radiation or particulate ionizing radiation irradiation method.

The method of further heating the water-absorbent resin composite comprises heating the water-absorbent resin composite at 100 to 250°C to polymerize the monomer remaining in the composite.

Regarding the method of adding a catalyst or a catalyst component capable of promoting the monomer polymerization to the water-absorbent resin composite solution, a reducing agent may be added to the water-absorbent resin composite since the radical generator may often remain in the composite when the monomer polymerization to generate the composite is effected in the presence of a redox-type polymerization initiator. The reducing agent may be any of sodium sulfite, sodium hydrogen sulfite, L-ascorbic acid or the like that is generally used in the redox-type polymerization initiator. In general, the reducing agent in the form of an aqueous 0.5 to 5 wt% solution thereof may be added to the water-absorbent resin composite. The amount of the reducing agent to be added is preferably in the range of 0.1 to 2 wt% on a dry resin weight basis. The reducing agent solution may be added to the composite by any appropriate method of spraying it on the composite with a sprayer, or of dipping the composite in the solution. The water-absorbent resin composite given the reducing agent is then heated so that the polymerizable monomer therein is polymerized. For example, it may be heated up to 100 to 150°C for about 10 to 30 minutes. Via the heating, the water content of the water-absorbent resin composite may be decreased. When the water content thereof is still high, however, the composite may be further dried in a drier to prepare an absorbent material product.

The method of ultraviolet irradiation to the water-absorbent resin composite may be done, using general UV lamp. The irradiation intensity, the irradiation time and the like vary, depending on the type of the fiber used, the amount of the remaining monomer immersed and the like. In general, the composite may be irradiated with a UV lamp at an intensity of 10 to 200 W/cm, preferably 30 to 120 W/cm, for an irradiation time of 0.1 seconds to 30 minutes. The distance between the lamp and the composite may be 2 to 30 cm. The water content of the water-absorbent resin composite then may be generally in the range of 0.01 to 40 parts by weight, preferably 0.1 to 1.0 part by weight, relative to one part by weight of the dry water-absorbent resin. The water content thereof smaller than 0.01 part by weight or the water content larger than 40 parts by weight unfavorably has a significant influence on the decrease of the remaining monomer. The atmosphere for UV irradiation may be in vacuum or may be in the presence of any of inorganic gases such as nitrogen, argon or helium, or in air. The irradiation temperature is not specifically defined. The purpose may be satisfactorily attained at room temperature. The UV-irradiation apparatus to be used is not also specifically defined. Any desired method may be used and includes for example, a method of UV irradiation on the composite while the composite is left to stand for a predetermined period of time; or a method of continuous UV irradiation on the composite on a belt conveyer.

In the method of radiation ray irradiation on the water-absorbent resin composite, high-energy radiation such as accelerated electron beam or gamma ray may be used. The irradiation dose varies, depending on the remaining monomer content in the composite, the water content of the composite and the like. The dose may be generally 0.01 to 100 Mrad and preferably 0.1 to 50 Mrad. At a dose larger than 100 Mrad, the water absorption potency is extremely decreased. At a dose less than 0.01 Mrad, further, a water-absorbent resin composite with higher water absorption potency and a larger water absorption speed but with an extremely small residual ratio of the remaining monomer can hardly be obtained. The water content of the water-absorbent resin composite then is appropriately selected, which is generally 40 parts by weight, preferably 10 parts by weight or less per one part by weight of the polymer. A water content exceeding 40 parts by weight is less effective for improving the water absorption speed, and disadvantageously influences significantly the decrease of the remaining monomer. The atmosphere for the irradiation of high-energy radiation on the composite may be in vacuum or may be in the presence of any of inorganic gases such as nitrogen, argon or helium, or in air. Preferably, the atmosphere is in air. When high-energy radiation irradiates the composite in air, the water absorption potency and water absorption speed may be greatly enhanced, while the residual monomer therein may be significantly decreased. The temperature for the irradiation is not specifically defined. The purpose can be sufficiently attained at room temperature.

The method (2) of converting the monomer into a different derivative includes, for example, a method of adding amine or ammonia to the water-absorbent resin composite and a method of adding thereto a reducing agent such as hydrogen sulfite salts, sulfite salts, or pyrosulfite salts.

The method (3) of removing the remaining monomer includes for example a method of extraction with an organic solvent and subsequent distillation and elimination of the organic solvent. In the method of extracting the monomer with an organic solvent, the water-absorbent resin composite is dipped in a water-containing organic solvent, to extract and remove the remaining monomer. As the water-containing organic solvent, for example, there may be used ethanol, methanol and acetone. The water content thereof is preferably in the range of 10 to 99 wt%, more preferably 30 to 60 wt%. In general, an organic solvent with a higher water content is at a higher elimination potency of the remaining monomer. When the water content of the organic solvent used is high, the energy consumption in the subsequent drying step increases. Generally, the time required for dipping the composite in the water-containing organic solvent may sufficiently be in the range of about 5 to 30 minutes. Preferably, a method of promoting the extraction of the remaining monomer, such as the shaking of the composite is employed. After dipping in the solvent, the composite is dried in a general drier.

As a method for distilling off the monomer residue, there includes a method of treating the composite in superheated steam or in a steam-containing gas. For example, saturated steam at 110°C is further heated up to 120 to 150°C to put the resulting superheated steam in contact with the composite, so that the remaining monomer in the thus-processed composite may be reduced. It is believed that during the evaporation of water in the water-absorbent resin in the form of steam, the remaining monomer may also be evaporated and removed from the water-absorbent resin by this method. According to the method, the removal of the remaining monomer from the water-absorbent resin composite can be done, concurrently with the drying of the resulting product.

(Surface crosslinking)

For the purpose of improving the water absorption potency thereof, the surface of the water-absorbent resin may be crosslinked with a crosslinking agent. It is generally known that the characteristic properties of resin particles can be improved by adding an appropriate amount of water together with a crosslinking agent to the surfaces of a powdery water-absorbent resin particle and subsequently heating the resin particles to crosslink the surfaces thereof. It is believed that as the consequence of the selective formation of a crosslinking structure on the surface, the shape can be maintained while swelling by absorbing water, with no inhibition of the swelling. At the step, first, a solution of a surface crosslinking agent is added to the water-absorbent resin composite. As the surface-crosslinking agent, for example, there can be used polyfunctional compounds copolymerizable with polymerizable monomers, such as N,N'-methylene-bis(meth)acrylamide and (poly) ethylene glycol di (meth) acrylate, or compounds having plural functional groups capable of reacting with a carboxylic acid group, such as (poly)ethylene glycol diglycidyl ether. In general, the amount of these surface-crosslinking agents may be used at 0.1 to 1 wt%, preferably 0.2 to 0.5 wt% of the water-absorbent resin composite. Preferably, the surface-crosslinking agents are used as a solution at a concentration of 0.1 to 1 wt%, preferably 0.2 to 0.5 wt% after dilution with water, ethanol or methanol, in order that the surface-crosslinking agents may be uniformly applied to the entire surface of the water-absorbent resin composite. Herein, desirably, the crosslinking agent solution is generally applicable by a method of spraying with a sprayer or a method of coating via a roll brush on the water-absorbent resin composite. After an excess amount of the crosslinking agent solution is applied to the water-absorbent resin composite, the composite is lightly squeezed between squeezing rolls or is blown with air to such a degree that the resin particle may not be crushed, to remove the excess crosslinking agent solution. The crosslinking agent solution may be applied to the water-absorbent resin composite at room temperature. The water-absorbent resin composite with the crosslinking agent solution applied is thereafter heated to progress the crosslinking reaction, to selectively form a crosslinking structure on the surface of the water-absorbent resin. The conditions for the crosslinking reaction may appropriately be determined depending on the crosslinking agent used. In general, the reaction is done at a temperature at 100°C or more for 10 minutes or more. In accordance with the invention, a crosslinked unsaturated carboxylic acid polymer is preferable as the water-absorbent resin. A crosslinked partially neutralized acrylic acid polymer is particularly preferable.

(Additives)

Various additives may be added to the water-absorbent resin composite or to the water-absorbent resin composite composition in order that the composite or its composition may have desired functions, depending on the intended use thereof. These additives include, for example, stabilizer for preventing polymer decomposition or deterioration due to liquids absorbed into the polymer, antimicrobial agents, deodorizers, deodorants, aromatic agents, and foaming agents.

Among them, a stabilizer for preventing polymer decomposition or deterioration owing to absorbed liquids is a stabilizer for preventing the water-absorbent resin from the decomposition or deterioration with excretes (e.g., human urine, feces) or body fluids (e.g., human blood, menstrual discharges, secretions). JP-A-63-118375 proposes a method of adding an oxygen-containing reducing inorganic salt and/or an organic antioxidant to polymer; JP-A-63-153060 proposes a method of adding an oxidizing agent to polymer; JP-A-63-127754 proposes a method of adding an antioxidant to polymer; JP-A-63-272349 proposes a method of adding a sulfur-containing reducing agent to polymer; JP-A-63-146964 proposes a method of adding a metal chelating agent to polymer; JP-A-63-15266 proposes a method of adding a radical chain reaction inhibitor to polymer; JP-A-1-275661 proposes a method of adding a phosphinic acid group or phosphinic acid group-containing amine compound or its salt to polymer; JP-A-64-29257 proposes a method of adding a polyvalent metal oxide to polymer; and JP-A- 2-255804 and 3-179008 propose a method of producing polymer in the presence of a water-soluble chain transfer agent. These are all applicable to the invention. In addition, the materials and the methods described in JP-A- 6-306202, 7-53884, 7-62252, 7-113048, 7-145326, 7-145263, 7-228788 and 7-228790 are all applicable to the invention. Concretely, for example, potassium oxalate titanate, tannic acid, titanium oxide, phosphinic acid amine (or its salts), phosphonic acid amine (or its salts) and metal chelates may be used in accordance with the invention. The stabilizers for human urine, human blood and menstrual discharges may be referred to as a human urine stabilizer, a human blood stabilizer, and a menstrual discharge stabilizer, respectively.

An antimicrobial agent may be used for preventing putrefaction with liquids absorbed. As the antimicrobial agent, there can be appropriately selected for example those introduced in "Sakkin Kokin Gijutsu No Shintenkai (Novel Development of Bactericidal and Antimicrobial Technique)", pp. 17-80 (by Toray Research Center (1994)); "Kokin Kokabizai No Kensa Hyokahou To Seihinsekkei (Methods of Examination and Evaluation of Antibacterial and Antifungal Agents., and Product Planning)", pp. 128-344 (by NTS (1997)); Japanese Patent 2,760,814; JP-A- 39-179114, 56-31425, 57-25813, 59-189854, 59-105448, 60-158861, 61-181532, 63-135501, 63-139556, 63-156540, 64-5546, 64-5547, 1-153748, 1-221242, 2-253847, 3-59075, 3-103254, 3-221141, 4-11948, 4-92664, 4-138165, 4-266947, 5-9344, 5-68694, 5-161671, 5-179053, 5-269164 and 7-165981.

The antimicrobial agent includes for example alkylpyridinium salts, benzalkonium chloride, chlorhexidine gluconate, pyridione zinc, and silver-containing inorganic powders. Typical examples of quaternary nitrogen-containing antibacterial agents are methylbenzethonium chloride, benzalkonium chloride, dodecyltrimethylammonium bromide, tetradecyltrimethylammonium bromide, and hexadecyltrimethylammonium bromide. Heterocyclic quaternary nitrogen-series antibacterial agents include dodecylpyridinium chloride, tetradecylpyridinium chloride, cetylpyridinium chloride (CPC), tetradecyl-4-ethylpyridinium chloride, and tetradecyl-4-methylpyridinium chloride.

Other preferred antibacterial agents include for example bis-guanides. These are described in detail for example in the specifications of USP 2,684,924, 2,990,425, 2,830,006 and 2,863,019. 1,6-Bis(4-chlorophenyl)diguanidohexane is the most preferred example of bis-guanides, which is known as chlorhexidine and its water-soluble salts. Especially preferred are hydrochloride, acetate and gluconate salts of chlorhexidine.

Some other types of antibacterial agents are also useful herein. For example, there are mentioned carbanilides, substituted phenols, metal compounds, and rare earth salts of surfactants. The carbanilides include 3,4,4'-trichlorocarbanilide (TCC, triclocarban) and 3-(trifluoromethyl-4,4'-dichlorocarbanilide (IRGASAN). One example of the substituted phenols is 5-chloro-2-(2,4-dichlorophenoxy)phenol (IRGASAN DP-300). The metal compounds include graphite and tin salts, for example, zinc chloride, zinc sulfide and tin chloride. The rare earth salts of surfactants are disclosed in EP-A 10819. Examples of the rare earth salts of the type are lanthanum salts of linear C10-18 alkylbenzenesulfonates.

Deodorants, deodorizers, and aromatic agents are used for preventing or reducing the unpleasant odor of the liquids absorbed. Such deodorants, deodorizers, and aromatic agents are introduced in, for example, "Atarashii Shoshu Dasshuzai To Gijutsu To Tenbo (Techniques and Views of New Deodorants and Deodorizers)", (by Toray Research Center (1994)); JP-A-59-105448, 60-158861, 61-181532, 1-153748, 1-221242, 1-265956, 2-41155, 2-253847, 3-103254, 5-269164 and 5-277143, and any of these are usable herein. Specifically, iron complexes, tea extracts and activated charcoal are exemplified as the deodorizer and deodorant. The aromatic agents include, for example, fragrances (e.g., citral, cinnamic aldehyde, heliotopin, camphor, bornyl acetate), wood vinegar, paradichlorobenzene, surfactants, higher alcohols, and terpene compounds (e.g., limonene, pinene, camphor, borneol, eucalyptol, eugenol).

A foaming agent or a foaming assistant may be added to the resin composite for allowing the water-absorbent resin to be porous and have an enlarged surface area so as to improve the water absorption potency of the water-absorbent resin. The foaming agent and the foaming assistant are introduced in, for example, "Gomu Purasuchikku Haigoyakuhin (Chemicals for Rubber and Plastics)" (by Rubber Digest, 1989, pp. 259-267), and any of these are usable herein. For example, there are mentioned sodium bicarbonate, nitroso compounds, azo compounds, sulfonyl hydrazide.

These additives may be appropriately added to the water-absorbent resin composite in any production stage of the water-absorbent resin composite, depending on the purpose, the action mechanism and the like. For example, the foaming agent may be added in the step of preparing the water-absorbent resin, preferably before the initiation of monomer polymerization or during the polymerization. The human urine stabilizer, the human blood stabilizer, the antimicrobial agent, the deodorants and the aromatic agents may be added in any step of producing the water-absorbent resin composite or producing the water-absorbent resin composite composition or even producing absorbent articles satisfactorily. They may be preliminarily added to fibers. Additionally, these additives may satisfactorily be added to constituents except the water-absorbent resin composite composing the absorbent article.

As described above, the water-absorbent resin composite composition can be produced by mixing the water-absorbent resin composite and a fiber together in a mixer. In such a manner, a water-absorbent resin composite composition with an appropriate composition of the water-absorbent resin composite and the fiber in mixture can be obtained. During the production, a water-absorbent resin particle never embedding any fiber therein or to which any fiber adheres may satisfactorily be mixed.

As the mixer, a solid mixer capable of powder-powder mixing, powder-fiber mixing or fiber-fiber mixing may be used. Specific examples of the solid mixer include for example those described in detail in "Kagaku Kogaku II (Chemical Engineering II)" (by Yoshitoshi Ohyama, Iwanami Zensho, 1963, p.229), which are rotary mixers such as cylindrical mixer, V-shaped mixer, double conical mixer, cubic mixer; and fixed mixers such as screw mixer, ribbon mixer, rotary disc mixer, fluidization mixer.

At the polymerization step of producing the water-absorbent resin composite, a water-absorbent resin composite composition may simultaneously be obtained by appropriately adjusting the feed position of the fiber and the like.

For the purpose of increasing the density of the water-absorbent resin composite composition to enhance the fixability of the fiber on the water-absorbent resin particle, the water-absorbent resin composite composition may satisfactorily be treated under compression. Using presses for example a tabular press or a roll press, conditions such as pressure, temperature and humidity may be adjusted appropriately to do the treatment. The pressure for the treatment under compression is within a range without any break of the water-absorbent resin particle. When the water-absorbent resin particle is broken, then, the broken particle pieces are released from the fiber and leaked from the final product water-absorbent article or the water-absorbent gel is dissociated or transferred from the fiber during swelling, so that the performance of the absorbent article is deteriorated finally.

When the water-absorbent resin composite composition is heated during the treatment under compression, the water-absorbent resin composite composition may be heated to a temperature of the melting point of the fiber used or less. When the water-absorbent resin composite composition is heated at a temperature of the melting point or more, then, the fiber fuses together to form a network structure. Thus, the function of the composite may possibly be deteriorated, disadvantageously. When humidification is to be done under the treatment under compression, generally, the humidification is done, using vapor. By appropriately selecting conditions for humidification, the density of the water-absorbent resin composite composition can be improved to enhance the fixability of the water-absorbent resin particle on the fiber.

The water-absorbent resin composite composition in accordance with the invention may readily be opened, because the individual constitutive components are independent of each other. For opening, the opening method described in the fiber description and the description about the water-absorbent resin composite may appropriately be used in the same way. However, an apparatus by which the water-absorbent resin cannot be broken via the mechanical impact or conditions therefor are preferably selected.

[Absorbent article]

(Constitution)

The absorbent article in accordance with the invention contains a water-absorbent resin composite comprising one water-absorbent resin particle and two or more fibers, where the water-absorbent resin particle is nearly spherical, and where one or more fibers of the two or more fibers described above are partially embedded in the water-absorbent resin particle and are partially exposed from the resin particle, and where one or more fibers of the two or more fibers are never embedded inside the water-absorbent resin particle but partially adhere to the surface of the water-absorbent resin particle.

The structure of the absorbent article in accordance with the invention may appropriately be determined, depending on the functions and use demanded toward the absorbent article. Typically, the absorbent article comprises a water-absorbent resin composite composition comprising the water-absorbent resin composite of the invention as the water-absorbent nucleus in appropriate combination with fluff, tissue, non-woven fabric, and polyolefin sheet.

So as to enhance the diffusibility of body fluids during use, a diffusion layer of a non-woven fabric comprising a hydrophobic fiber such as polyethylene fiber, polypropylene fiber and polyester fiber may satisfactorily be used in so-called paper diaper and sanitary napkin. The water-absorbent composite may be used freely in mixture with fluff pulp and the like.

Powdery water-absorbent resins such as those commercially available may also be mixed into the water-absorbent resin. For mixing, such water-absorbent resins may be mixed and used within a range such that the dropout ratio of the resulting water-absorbent resin as measured according to the method for measuring the dropout ratio of water-absorbent resin as described below may preferably be 5% or less.

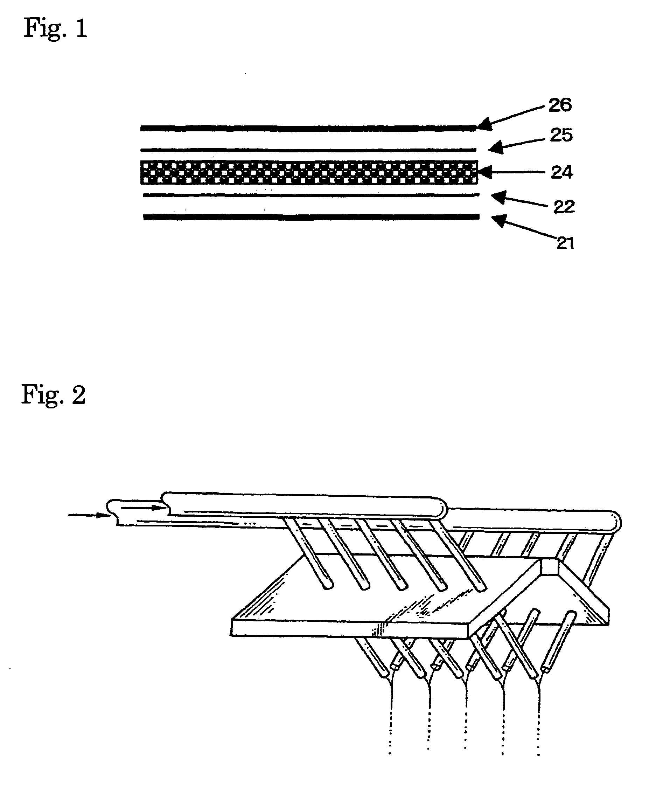

As to a typical constitution example of a diaper as the absorbent article, reference is made to the following Examples and Fig. 1. The structure in Fig. 1 shows a laminate of tissue 22, a highly densified water-absorbent resin composite composition 24, tissue 25 and a non-woven fabric comprising a water-permeable polyester fiber 26 in this order on the water-impermeable polyethylene sheet 21. After the preparation of the laminate, the individual layers closely adhere together under pressure. After pressure release, four sides are fused together under heating, to prepare an absorbent article. Aqueous liquids to be absorbed are absorbed from the side of the non-woven fabric comprising the water-permeable polyester fiber 26 into the water-absorbent resin composite composition 24.

Aqueous liquids can be absorbed immediately by arranging fibrous materials such as the tissue 25 and the non-woven fabric comprising the water-permeable polyester fiber 26 on the top of the water-absorbent resin composite composition 24, as shown in the structure of Fig. 1. Additionally, the absorbent article can be designed in such a manner that the absorbed aqueous liquids may hardly be released even when a pressure is loaded on the absorbent article.

By additionally inserting a material providing bulkiness to the absorbent article, such as fluff pulp, skin touch can be improved, to widen the applicability to bodies. The coating weight of the material providing the bulkiness is preferably 80 to 250 g/m$^2$, more preferably 100 to 220 g/m$^2$. The material providing the bulkiness is preferably arranged in between the water-absorbent resin composite composition 24 and a base material such as water-impermeable polyethylene sheet 21. The material may satisfactorily be arranged in such a manner that the material sandwiches the water-absorbent resin composite composition 24 from the top and the bottom. In the case of sandwiching from the top and the bottom, the material on the lower side may preferably have a larger coating weight.

(Thinner preparation)

For producing an absorbent article of a thinner type, preferably, the water-absorbent resin composite composition in accordance with the invention is preliminarily prepared into a thinner type. Specifically, the bulk density of the water-absorbent resin composite composition in accordance with the invention is within a range of preferably 0.20 to 1.10 g/cm$^3$, more preferably 0.20 to 0.85 g/cm$^3$. The density after the preparation thereof into a thinner type may be adjusted by conditions for pressurization, heating and humidification of the water-absorbent resin composite composition. The thickness after the preparation into a thinner type is preferably 0.2 to 20 mm, more preferably 0.2 to 10 mm, still more preferably 0.2 to 5 mm.

As a method for preparing the water-absorbent resin composite composition into a thinner type, for example, a method including pressurizing the water-absorbent resin composite composition using presses. As the presses, for example, a tabular press or a roll press can be used. The pressure for pressurization is within a range never causing any break of the water-absorbent resin particle. When the water-absorbent resin particle is broken, then, the broken particle pieces are released from the fiber and then leaked from the absorbent article or the water-absorbent gel is dissociated or transferred from the fiber during swelling, so that the performance of the absorbent article is deteriorated finally.

Other than the pressurization, the water-absorbent resin composite composition may be treated with heating or humidification, if necessary. When the water-absorbent resin composite composition is to be heated, a temperature of the melting point or less is selected as the heating temperature. When the water-absorbent resin composite composition is heated at a temperature of the melting point or more, then, the fiber fuses together to form a network structure. Thus, a result different from the object of the invention emerges. When humidification is to be done, a method including spraying water on the water-absorbent resin composite composition or a method including feeding water in vapor may be used. The humidification level may appropriately be selected, depending on the content of the water-absorbent resin particle or the like. Generally, the water to be used for humidification is at an amount of 500 g or less per 1 m$^2$, preferably 300 g or less per 1 m$^2$, more preferably 100 g or less per 1 m$^2$. In the case that the humidification level is too high, the water-absorbent resin particle is softened and crushed, or the fiber fuses together to form a network structure. Thus, a result different from the object of the invention emerges. Additionally, a large amount of water should be distilled off subsequently. Therefore, it is not economical. In the case of feeding water in vapor, the vapor pressure is lower than 10 MPa. Preferably, the vapor pressure is lower than 1 MPa. The velocity of vapor feed may appropriately be selected, depending on the content of the water-absorbent resin particle, the time needed for the humidification treatment and the like. The velocity is generally 300 kg/hr or less per 1 m$^2$, preferably 100 kg/hr or less per 1 m$^2$, more preferably 50 kg/hr or less per 1 m$^2$. Additionally, the time needed for the treatment is generally one hour or less, preferably 30 minutes or less, still more preferably 20 minutes or less. When the amount of vapor fed is too much, the water-absorbent resin absorbs water, so that the resin is softened or crushed or the fiber fuses together to form a network structure. Thus, a result different from the object of the invention emerges. Additionally, a large amount of water should be distilled off subsequently. Therefore, it is not economical. Alternatively, humidification may also be carried out by moisten the absorbent article with a sprayer.

Additionally, the water-absorbent resin composite composition may also be applicable to the use of sheet-like absorbent materials proposed in water-absorbent sheets described as in JP-A-63-267370, 63-10667, 63-295251, 63-270801, 63-294716, 64-64602, 1-231940, 1-243927, 2-30522, 2-153731, 3-21385, 4-133728 and 11-156118.

(Dropout ratio of water-absorbent resin)

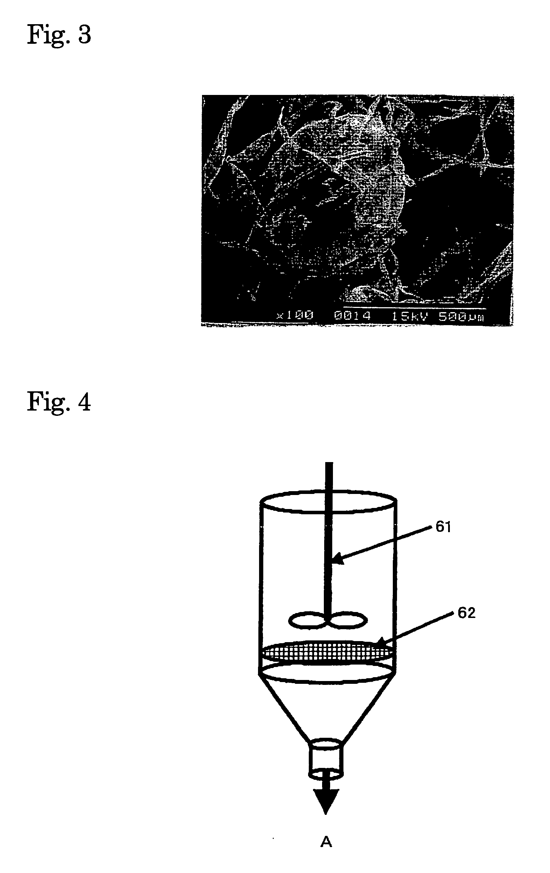

When the absorbent article of the invention is shaken at a vibration number of 165 vibrations/minute and a rotation number of 290 rotations/minute for 60 minutes, using a shaker shown in the Fig. 4 of JIS Z-8815, the weight of the water-absorbent resin particle dissociated from the water-absorbent article is preferably at 5% by weight or less of the total weight of the water-absorbent resin in the water-absorbent article before shaking. About the details of the test, the following description of the dropout ratio of the water-absorbent resin and JIS Z-8815 can be referred to.

The dropout ratio of the water-absorbent resin in the absorbent article in accordance with the invention is preferably 5% or less, more preferably 3% or less, still more preferably 1% or less. When the dropout ratio of the water-absorbent resin exceeds 5%, the water-absorbent resin particle dissociated from the absorbent article is locally present via vibration or the like, so that regions in the absence of any water-absorbent resin are generated. Accordingly, liquids cannot be sufficiently absorbed, causing leakage. When an absorbent article is produced, using the water-absorbent resin composite composition, generally, an absorbent article at a dropout ratio of the water-absorbent resin of 5% or less can be obtained. When a water-absorbent resin article is to be further added to the absorbent article, the amount of the water-absorbent resin particle added is at 5% or less of the total weight of the water-absorbent resin. When an absorbent article is to be produced by a method including a pressurization step, preferably, pressurization is done at a level such that the water-absorbent resin particle may not be crushed. Even when the water-absorbent resin particle is crushed into a freely transferable water-absorbent resin particle, the amount of the water-absorbent resin particle is preferably adjusted to 5% or less of the total weight of the water-absorbent resin.

(Uses)

The absorbent article of the invention can preferably be used for example in sanitary materials such as paper diapers for babies and toddlers, paper diapers for adults, pads for incontinence and sanitary napkins, industrial materials such as absorption sheets for liquid waste, holding sheets, coolers, water retentive materials, sealing materials, and dew-preventing agents for buildings, and agricultural materials such as water retentive agents for soil, water retentive sheets for growing seedlings, and freshness keepers of vegetables, and water retentive agents.

Second invention

The absorbent article in accordance with the second invention contains a water-absorbent resin particle and a fiber, and comprises a water-absorbent resin composite composition, where the restoring ratio represented by the aforementioned formula (1) is 50% or less. Any absorbent article meeting the conditions may be in accordance with the second invention, with no specific limitation as to the constitutive materials of the second invention or the details of the structure.

Because the restoring ratio of the water-absorbent resin composite composition in the absorbent article in accordance with the second invention is 50% or less, the thickness of the absorbent article even after pressurization into a thinner type never increases greatly. When the absorbent article of a thinner type is packaged as a commercial product, accordingly, the package is never broken via the restoring force or the thickness is never increased excessively even when an individual user opens the package. Because the thickness is not greatly increased, the wear touch is never deteriorated. The absorbent article comprising the water-absorbent resin composite composition at a restoring ratio of 50% or less in accordance with the second invention is highly practical. The restoring ratio of the water-absorbent resin composite composition is preferably 45% or less, still more preferably 40% or less.

The water-absorbent resin composite composition at a restoring ratio of 50% or less can be produced by selecting a structure where the restoring force of a fiber may be smaller. In absorbent articles in the related art where non-fabricated fibers are bonded together through a water-absorbent resin to form a three-dimensional fiber network, the restoring force of the fibers binding the water-absorbent resin together is loaded under pressurization. By selecting a structure where fibers can hardly form such a three-dimensional network, in accordance with the second invention, the restoring ratio can be adjusted to 50% or less. When a water-absorbent resin composite satisfying the conditions in accordance with the first invention is used, an absorbent article satisfying the conditions in accordance with the second invention can be provided.

As to the method for measuring the restoring ratio of water-absorbent resin composite compositions, the following description about the measurement of restoring ratio can be referred to.

Third invention

The absorbent article in accordance with the third invention contains a water-absorbent resin particle and a fiber, where the gel dropout ratio represented by the formula (2) is 10% or less. Any absorbent article meeting the conditions may be satisfactory in accordance with the third invention, with no specific limitation as to the constitutive materials of the third invention or the details of the structure.

Because the gel dropout ratio in the absorbent article in accordance with the third invention is 10% or less, possibly, the gel detachment from the fiber during swelling can be prevented so that the transfer of the water-absorbent gel and the leakage of liquids from the absorbent article are further prevented. A problem of the leakage of liquids once absorbed in sanitary materials such as diaper, in particular, when loaded with pressure or vibration after the liquids are absorbed, has been remarked in the related art. In accordance with the third invention, however, that problem can be reduced greatly. Consequently, an absorbent article with good wear touch and high shape stability over a long period of time can be provided. The absorbent article with a gel dropout ratio of 10% or less in accordance with the third invention is highly practical. The gel dropout ratio of the absorbent article is preferably 8% or less, more preferably 5% or less.

The absorbent article with a gel dropout ratio of 10% or less can be produced by selecting a structure where the fiber is hardly detached from the gel during swelling.. When a water-absorbent resin composite satisfying the conditions in accordance with the first invention is used, an absorbent article satisfying the conditions in accordance with the third invention can be provided.

As to the method for measuring the gel dropout ratio of water-absorbent articles, further, the following description about the measurement of gel dropout ratio is referred to.

Fourth invention

The absorbent article in accordance with the fourth invention comprises a water-absorbent resin composite with a pliability of 5.0 to 9.5 cm as measured according to the heart loop method defined in JIS L-1096. Any absorbent article satisfying the conditions may be satisfactory, with no specific limitation as to the details of the constitutive materials and structure of the fourth invention.

Because the absorbent article of the fourth invention comprises the water-absorbent resin composite composition of a pliability of 5.0 to 9.5 cm, the absorbent article has appropriate flexibility. When the absorbent article is therefore processed into a diaper, for example, the diaper can fit buttocks appropriately, to give great wear touch. When the pliability exceeds 9.5 cm, unpreferably, the resulting absorbent article is too soft to retain the shape thereof. When the pliability is less than 5.0 cm, the softness is so poor that the resulting diaper cannot fit body parts such as buttocks, which have variable shapes individually. The pliability of the absorbent article is preferably 5.5 to 9.5 cm, more preferably 6.0 to 9.5 cm, still more preferably 7.0 to 9.5 cm.

The absorbent article comprising the water-absorbent resin composite composition with a pliability of 5.0 to 9.5 cm can be provided by selecting as the structure thereof a structure where the fiber never forms a three-dimensional network and which is nearly spherical so that the water-absorbent resin slips when the resin collides with each other. When a water-absorbent resin composite satisfying the conditions for the first invention is used, for example, an absorbent article satisfying the conditions for the fourth invention can be provided.

As to the method for measuring the pliability of absorbent article, reference is made to the following description about the measurement of pliability and JIS L-1096.

The invention is described more concretely with reference to the following Examples, Comparative Example and Test Example. The materials, the reagents, the ratios, the procedures and the like as described below may be modified without departure from the spirit and scope of the invention. Accordingly, the scope of the invention should not be construed in a limitative way based on the specific examples described below.

In the following descriptions, "composite A", "composite B" and "composite C" mean water-absorbent resin composites with the structures defined below, respectively.

1) Composite A: A water-absorbent resin composite comprising one water-absorbent resin particle and two or more fibers, where one or more of the two or more fibers are partially embedded inside the resin particle and are partially exposed from the resin particle and where one or more of the two or more fibers are never embedded inside the resin particle but partially adhere to the surface of the resin particle.

2) Composite B: A water-absorbent resin composite comprising one or more water-absorbent resin particles and one or more fibers, where the one or more fibers are partially embedded inside the resin particles and are partially exposed from the resin particles and where none of the fibers adheres to the surface of the resin particles.

3) Composite C: A water-absorbent resin composite comprising one or more water-absorbent resin particles and one or more fibers, where the one or more fibers partially adhere to the surface of the resin particles and none of the fibers is partially embedded inside the resin particles.

<Production Example 1>

Production of water-absorbent resin composite

3.3 parts by weight of water were added to 100 parts by weight of acrylic acid, to which 133.3 parts by weight of an aqueous 25 wt% solution of sodium hydroxide were added under cooling at 25°C or lower to prepare an aqueous partially neutralized acrylic acid solution with a monomer concentration of 50 wt% and a degree of neutralization of 60 mol%.

Then a solution A was prepared by adding 0.14 part by weight of N,N'-methylene-bisacrylamide as a crosslinking agent, and 4.55 parts by weight of an aqueous 31 wt% hydrogen peroxide solution as an oxidizing agent, to 100 parts by weight of the aqueous partially neutralized acrylic acid solution.

Separately, a solution B was prepared by adding 0.14 part by weight of N,N'-methylene-bisacrylamide as a crosslinking agent, and 0.57 part by weight of L-ascorbic acid as a reducing agent, to 100 parts by weight of the same aqueous partially neutralized acrylic acid solution.

The solution A and the solution B, thus prepared, were mixed together through the nozzles shown in Fig. 2. The nozzles in. Fig. 2 are of an inner diameter of 0.125 mm, and five nozzles for each solution are arranged at an interval of 1 cm. The cross angle between the solution A and the solution B flowing out of the nozzles was adjusted to 30 degrees, while the distance between the nozzle tips was adjusted to 4 mm. The solution A and the solution B were individually heated at a liquid temperature of 40°C, and were then fed via pumps so that the flow rate of each solution could be 5.4 m/sec.

The solution A and the solution B were combined together immediately after the solutions were out of the nozzles of the individual nozzle pairs, to form individual liquid columns of about 10 mm in length. Thereafter, the liquid columns fell down in the form of liquid droplets in a gas phase (in air at a temperature of 50°C), in the progress of polymerization. An opened fiber pulp (average length of 2,500 $\mu$m, average width of 2.2 dtex and a contact angle with water being 0°) was fed at a rate of 23 g/m in the form of a mix phase flow with air (fiber : air = 1:100) at a linear velocity of 10 m/s, intermediately in the fall of the liquid droplets (at a polymerization ratio of 40%), which was located 1.6 m below the nozzle tips. The liquid droplets collided with each other and were then mixed with the pulp fiber in the gas phase, to form a composite, which was deposited on a polyester net arranged 3 m below the nozzle tips.

The deposit was recovered, dried, and sieved, from which the fiber in no contact with the water-absorbent resin (fiber never embedded in or adhering to the water-absorbent resin; referred to as "free fiber" hereinbelow) was removed, to obtain a product comprising the water-absorbent resin and the fiber.

Under microscopic observation of the product, it was confirmed that the product was a composition comprising the composite A (the water-absorbent resin particle being approximately spherical; Fig. 3 shows a scanning microscopic photograph thereof) and the composite C (the water-absorbent resin particle being approximately spherical). No water-absorbent resin particle embedding the fiber therein or being bonded with the fiber was observed.

<Production Examples 2 to 6 and Production Example 13>

Production of water-absorbent rein composites

Products comprising water-absorbent resin composites were produced in the same manner as in Example 1, except for the modification of the fiber material, the average fiber length and the average fiber size as shown in Table 1.

In Production Example 5, polyethylene terephthalate (PET) fiber with a fiber diameter of 1.7 dtex, a length of 0.9 mm and a contact angle with water being 80° was used. In Production Example 6, a fiber mixture of a nylon type with a fiber diameter of 1. 7 dtex, a fiber length of 0.9 mm and a contact angle with water being 50° and a rayon type with the same fiber diameter and length but with a contact angle with water being of 0° at a ratio of 1:1 was used. In Production Example 13, a fiber mixture of a polypropylene type (PP) with a fiber diameter of 1.5 dtex, a fiber length of 2.5 mm and a contact angle with water being 90° and a polyethylene type (PE) with the same fiber diameter and length but with a contact angle with water being of 90° at a ratio of 1:1 was used.

Under microscopic observation of the product of Production Example 13, it was confirmed that the product never contained the composite A.

<Production Example 7>

Production of water-absorbent resin composite

According to an Example of JP-A-63-63723, a water-absorbent resin composite was produced by the following procedures.

In a 200-ml beaker, 45.0 g of acrylic acid and 1.5 g of distilled water were placed. The mixture was neutralized with 60. 0 g of an aqueous 25% sodium hydroxide solution under cooling at 35°C or lower, to prepare an aqueous partially neutralized acrylic acid solution (at a monomer concentration of 50 wt% and a neutralization degree of 60 mol%). 41.9 mg of N, N' -methylene-bisacrylamide and 0.31 g of L-ascorbic acid were dissolved in the aqueous partially neutralized acrylic acid solution, to obtain an aqueous mix monomer solution.

The upper face of a 300-ml stainless-steel beaker was completely sealed with a polyester sheet. Then, the upper sheet was punctured. A rubber tube was inserted into the resulting hole, to substitute sufficiently the inside atmosphere of the beaker with nitrogen. The aqueous mix monomer solution was poured into the stainless-steel beaker, and the beaker was then immersed in a water bath at 50°C. With stirring, 0.84 g of aqueous 30% hydrogen peroxide was added thereto, for polymerization. About one minute later, the mixture reached the highest temperature of 110°C.

After the mixture was retained at its immersed state in the warm water bath at 50°C for 2 hours, the mixture was then cooled to 20°C, to obtain a hydrous water-absorbent resin. 70 g of the resulting hydrous water-absorbent resin (the water-absorbent resin of 35 g) was mixed and kneaded with 200 g of water and 10 g of the same pulp as used in Production Example 1 in a screw-rotary mixer for about 2 hours. The resulting mixture was then dried in a drier under reduced pressure at 100°C for 8 hours, and was further ground in a rotation blade-type grinder. The resulting powder was sieved, from which free fibers were removed, to obtain a product comprising a water-absorbent resin and the fiber.

The product was observed with a microscope. The composite B could be confirmed. However, none of the composites A and C could be confirmed.

<Production Example 8>

Production of water-absorbent resin composite

By the same method as for the procedures in Production Example 1 except for the following modifications that the pulp fiber was fed along with air at a feed rate of 23 g/minute into a position 0. 8 m below the nozzle tips (at a polymerization ratio of 15%) and the collection was done on a polyester net at a position 1.6 m below the nozzle tips (at a polymerization ratio of 40%), a product comprising a water-absorbent resin composite was obtained.

Under microscopic observation of the product, it was confirmed that the product comprised the composite B. It was additionally confirmed that the composite was in a sheet-like structure where the fiber formed a three-dimensional network via the water-absorbent resin particle. Meanwhile, none of the composites A and C was found.

<Production Example 9>

Production of water-absorbent resin composite

By absolutely the same method as in the procedures in Production Example 1 except for the feeding of the fiber from the fiber feed port arranged 0.8 m below the nozzle tips, a water-absorbent composite was produced, to obtain a product comprising the water-absorbent resin composite.

Microscopic observation of the product indicated that the composition comprised the composite A (the water-absorbent resin particle being approximately spherical) and the composite B.

<Production Example 10>

Production of water-absorbent resin composite

47.5 parts by weight of the composition obtained in Production Example 3, 47.5 parts by weight of the composition comprising the two types of the water-absorbent resin composites obtained in Production Example 9, and 5 parts by weight of the same fiber as used in Production Example 1 were uniformly mixed together with a rotation blade-type mixer, to obtain a product.

Under microscopic observation of the product, it was confirmed that the product was a composition comprising the composite A (the water-absorbent resin particle being approximately spherical), the composite B, the composite C and free fiber. As a result of the microscopic observation, the weight ratio of the composite A in the total weight was 0.24.

<Production Example 11>

Production of water-absorbent resin composite

A water-absorbent resin composite was produced by the following procedures according to an Example in JP-A-11-93073.

125 parts by weight of an aqueous 80 wt% acrylic acid solution and 133 parts by weight of an aqueous 30 wt% sodium hydroxide solution were mixed together to prepare an aqueous partially neutralized acrylic acid solution at a neutralization degree of 72 mol% and a concentration of 47% by weight. To the aqueous partially neutralized acrylic acid solution was added a solution of 0.04 part by weight of a crosslinking agent, N,N'-methylene-bisacrylamide and 0.3 parts by weight of an initiator, 2,2'-azobis(2-amidinopropane) dihydrochloride dissolved in 13 parts by weight of distilled water. Then the resulting solution was degassed with nitrogen, to prepare an aqueous monomer solution.

In place of the nozzle used in Example 1, herein, a single solution-type spray nozzle was used. Through the nozzle, the monomer solution was fed using a pump to a flow rate of 40 ml/minute, while the solution temperature was kept at 25°C. In the progress of polymerization, the monomer solution dropped down in the form of liquid droplets in a gas phase (in air at a temperature of 25°C) . On the other hand, the same fiber as used in Production Example 5 was fed at a rate of 23 g/minute in the mix phase flow with air (fiber : air = 1:100) at a linear speed of 10 m/second, intermediately in the falling of the liquid droplets at a position located 0.8 m below the nozzle tips (polymerization ratio < 1%) . The liquid droplets collided with the fiber in the gas phase, to form a composite, which was deposited 3 m below the nozzle tips. The deposit was recovered and placed in an oven at 80°C, to promote the polymerization of the deposited aqueous monomer solution for 30 minutes. Subsequently, the resulting product was treated in hot air at 140°C, to obtain a recovered product comprising the dried water-absorbent resin composite.

Then, the recovered product was sieved. Then it was attempted to remove free fibers in no contact with the water-absorbent resin. However, the water-absorbent resin also functioned as an adhesive for the fiber. In other words, the fiber was in a sheet-like structure where the fiber formed a three-dimensional network via the water-absorbent resin particle. Therefore, no free fibers existed actually. A product comprising the water-absorbent resin and the fiber could be obtained in such a manner.

Under microscopic observation of the product, a structure was confirmed, where a part of the fiber adhered to the surface of the resin particle. However, no structure was found, where a part of the fiber was embedded in the water-absorbent resin.

<Production Example 12>

Production of water-absorbent resin composite

A highly water-absorbent resin was extracted from 42 L-size diapers commercially available from Uni-Charm, under the trade name of Mooney Sara-sara Cotton (Lot No. 921,411,943). 70 parts by weight of the extracted highly water-absorbent resin and 30 parts by weight of a pulp fiber (average length of 2, 500 $\mu$m, average width of 2.2 dtex and a contact angle of 0°) were introduced onto the 60-mesh metal net 62 of a pulp mixer shown in Fig. 4. While aspirating the mesh metal net along the direction A to a 70-mmHg pressure difference between the upper and bottom sides of the mesh metal net, the agitation wing 61 was rotated for mixing the resin and the pulp fiber together, to prepare a composition comprising the water-absorbent resin and the fiber.

Under microscopic observation of the composition, it was confirmed that the composition never contained the composite A.

<Production of highly densified water-absorbent resin composite composition>

Using the weight ratios of the individual water-absorbent resin composites produced in the Production Examples and the dry weight ratios of the binding fibers and the water-absorbent resins composing the individual water-absorbent composites, water-absorbent resin composites and free fibers were mixed together in such a manner that the coating weight of the water-absorbent resins and the dry weight ratio of the fiber (binding fiber + free fiber) and the water-absorbent resin might have given values.

For preparing a highly densified water-absorbent resin composite composition with a coating weight of water-absorbent resin being "P" [g/m$^2$] and a dry weight ratio "F" [w/w] of free fiber and water-absorbent resin from a water-absorbent resin composite "x" [g/m$^2$] and free fiber "y" [g/m$^2$], where the dry weight ratio of composite A, composite B and composite C was a, b and c, respectively (a + b + c = 1) and the dry fiber weight ratio composing the individual composites was $\alpha$, $\beta$ and $\gamma$, respectively, the following equations are established:

$$\{a(1 - \alpha) + b(1 - \beta) + c(1 - \gamma)\}x = P \ [g/m^2],$$

and

$$y/[\{a(1 - \alpha) + b(1 - \beta) + c(1 - \gamma)\}x] = F \ [w/w].$$

When a, b, c, $\alpha$, $\beta$, $\gamma$, P and F are given in these equations, accordingly, x and y can be calculated. Herein, P = 300 g/m$^2$ (constant value).

The mixture was uniformly spread closely on a stainless-steel plate to a size of 40 cm $\times$ 10 cm. An additional stainless-steel plate was overlaid on it. A load of 0.6 MPa was applied from both the sides. Then, the plate was left to stand as it was for 20 minutes. Then, the pressure was released, to obtain a high-density water-absorbent resin composite composition.

<Examples and Comparative Examples>

Production of absorbent articles

Using the highly densified water-absorbent resin composite composition, 18 types of diapers as absorbent articles were produced by the following procedures (Examples 1 to 13 and Comparative Examples 1 to 5).

(1) Tissue 22 (with coating weight of 14 g/m$^2$), high-density water-absorbent resin composite composition 24 (at an amount to 300 g/m$^2$ water-absorbent resin and of a size of 10 cm × 40 cm), tissue 25 (with coating weight of 14 g/m$^2$), and non-woven water-permeable polyester fiber fabric 26 (with coating weight of 23 g/m$^2$) were piled up in that order on a water-impermeable polyethylene sheet 21 (with coating weight of 18 g/m$^2$), as in Fig. 1. This was sandwiched between a pair of stainless-steel plates and kept under a load of 0. 6 MPa for 20 minutes, to compact the layers.
(2) The pressure was released. Then, the four edges of the water-absorbent article were heat-sealed.
(3) The heat-sealed outer edges were then trimmed, to obtain a water-absorbent article of a size of about 10 cm × about 40 cm.

In Example 10, however, distilled water was sprayed to 10 g/m$^2$, before overlaying the stainless-steel plate. Then, a pressure of 0.6 MPa was loaded for the processing over 20 minutes.
In Example 11, a stainless-steel box with 1-mm$\phi$ holes punctured at an equal interval was overlaid on the stainless-steel plate facing the water-absorbent resin composite composition. Subsequently, a pressure of 0.6 MPa was loaded, to feed steam at 150°C at 50 kg/hr/m$^2$ into the stainless-steel box for treatment for 20 minutes.

<Test Example>

Assessment of water-absorbent resin composites, highly densified water-absorbent resin composite compositions and absorbent articles

The water-absorbent resin composites produced in Examples 1 to 13, and Comparative Examples 1 to 5 were observed of their shapes, and were measured of the average particle diameters of the water-absorbent resins, the dry weight ratios of the individual composites, the dry weight ratios of binding fibers and water-absorbent resins composing the individual composites, the water retentive capacities and the water-absorbent rates. Concerning the highly densified water-absorbent resin composite compositions prepared from the individual water-absorbent resin composites and free fibers, the thickness, the bulk density, the pliability, the restoring ratio and the opening property thereof were measured and evaluated. Herein, it was assumed that the weight ratios of the individual composites and free fibers composing the highly densified water-absorbent resin composite compositions and the dry weight ratios of the free fibers and the water-absorbent resins were never modified by the treatment for preparing the highly densified products. Further, absorbent articles were prepared using the highly densified water-absorbent composite compositions, to measure the absorption rates, the amounts of released water, the dropout ratios of water-absorbent resins, and the gel dropout ratios thereof. The individual measurement results and evaluation results are shown in Table 1.
Additionally, the methods for measuring the individual physical properties and the evaluation methods in accordance with the invention are now described below.

1. Fibers

1-1) Contact angle with water

(1) The fibers were dissolved or dispersed in a solvent capable of dissolving or dispersing the fibers, to prepare solutions at a concentration from 1 to 10% by weight.
(2) The solutions were spread on a thin layer on a petri dish; the solvent was gently distilled off in dry air at room temperature; and the resulting products were sufficiently dried, to prepare thin film-like molded articles on the dish.
(3) The contact angle of the atmospheric surface of the film-like molded articles with distilled water was determined at 25°C. The contact angle was measured with an automatic contact angle meter, Kyowa Kaimen Kagaku's Model CA-V.

1-2) Spatial density

On the assumption that the fibers fed into a reactor could move downward along with the air stream fed into a reactor as a mix phase flow, the amount of the fibers retained in the reaction field was calculated. The amount thereof retained was divided by the volume of the overall reaction field, to calculate the spatial density of the fibers in the reaction field.

2. Liquid droplets

2-1) Liquid droplet diameter

The average particle size "dp" and the monomer concentration "Cm" of the water-absorbent polymer particle composing the water-absorbent resin composite were measured according to the method 3-2) described hereinbelow, to calculate the liquid droplet diameter according to the following formula.

$$\text{Liquid droplet diameter "dd"} = dp/(Cm)^{1/3}$$

2-2) Spatial density

On the assumption that the liquid droplets would fall downward in a reaction field at an initial speed of the downward ejection speed from the nozzle, the amount of the liquid droplets retained in the reaction field was calculated. Then, the retention amount was divided by the volume of the overall volume of the reaction field, to calculate the spatial density of the liquid droplets in the reaction field.

2-3) Polymerization ratio (polymerization ratio at position in contact with fibers)

(1) A beaker charging about 150 g of methanol therein was arranged in such a manner that the liquid face of methanol might be positioned at the position for fiber introduction, while liquid droplets of a reaction mixture after polymerization started were formed. In the progress of polymerization, about 1 g of the liquid droplets were allowed to fall into methanol in the beaker.
(2) The amount of the monomer in methanol was measured by liquid chromatography.
(3) The polymer in methanol was dried at 130°C under reduced pressure for 3 hours, to measure the weight.
(4) Based on the individual weights, the polymerization ratio was calculated according to the following equation ("Mp" represents polymer weight and "Mm" represents monomer weight).

$$\text{Polymerization ratio (\%)} = [Mp/(Mn + Mp)] \times 100$$

3. Water-absorbent resin composite

3-1) Identification of morphology of water-absorbent resin composite

(1) Under observation of the water-absorbent resin composite with a scanning electron microscope under a magnification $\times$ 20 to $\times$ 20,000, a structure where the fiber was partially embedded inside the resin and the fiber was partially exposed from the resin was confirmed. Otherwise, the adhesion status of the fiber to the resin surface was confirmed.
(2) Furthermore the water-absorbent resin composite was continuously cut with a precision cutter such as microtome in the cross-section. The resulting cross sections were observed with a magnification $\times$ 20 to $\times$ 20, 000. A structure was confirmed, where the fiber was partially embedded inside the resin and the fiber was partially exposed from the resin. Otherwise, the adhesion status of the fiber to the resin surface was confirmed.

3-2) Average particle size of water-absorbent resin particle

Water-absorbent resin composites were photographed with an optical microscope. Then, 100 water-absorbent resin particles (any of the water-absorbent resin particles as subjects for the measurement in this specification were all nearly spherical) were appropriately selected, to measure the diameters thereof. The number-average diameter was defined as average particle size.

3-3) Dry weight ratio of individual water-absorbent resin particles

With an optical microscope, about 1 g of a water-absorbent resin composite was classified in composite A, composite B and composite C. The weights of the individual composites were measured with a precision balance, to calculate the dry weight ratio of the individual water-absorbent composites.

3-4) Dry weight ratio of binding fiber and water-absorbent resin composing each composite

From each of the water-absorbent resin composites classified above in the item 3-3) the fiber therein was isolated using a chemical agent selectively decomposing the water-absorbent resin in the composites, to measure the weight of the fiber to determine the dry weight ratio.
Specifically, for example, the water-absorbent resin composite A was determined of the dry weight ratio as follows.

(1) The weight of the water-absorbent resin composite A determined in the item 3-3) was defined as Wc. The water-absorbent resin composite A was placed in a sealable 50-ml glass bottle. Then, a solution of 0.03 g of L-ascorbic acid dissolved in 25 g of distilled water was added to swell the water-absorbent resin composite. The resulting resin composite was left to stand at 40°C for 24 hours.
(2) The content of the glass bottle was filtered through a filter paper dried under reduced pressure at 80°C for 3 hours to a constant weight, under suction with an aspirator at an ultimate vacuum level of 10 to 25 mmHg. The fiber remaining on the filter paper was sufficiently washed with water, dried at 100°C for 5 hours and then accurately weighed. Thus, the weight was defined as Wf.
(3) According to the following equation, the dry weight ratio of the binding fiber to the water-absorbent resin composing the water-absorbent resin composite A was obtained.

```
Dry Weight Ratio of binding fiber to water-absorbent resin
= Wf/(Wc - Wf)
```

3-5) Water retentive capacity

(1) A necessary amount of physiological saline (aqueous 0.9 wt% sodium chloride solution) was preliminarily prepared.
(2) The ratio of the biding fiber to the water-absorbent resin in the water-absorbent resin composite was determined by the same method as in the above item 3-3). Then, the water-absorbent resin composite was collected so that the weight of the water-absorbent resin in the composite might be about 1 g. The weight was defined as W1. In addition, the weight (W2) of the fiber in the water-absorbent resin composite was calculated on the basis of the ratio of the fiber to the water-absorbent resin.
(3) The water-absorbent resin composite was placed in a 250-mesh nylon bag (20 cm × 10 cm) and dipped in 500 ml of physiological saline at room temperature for 30 minutes.
(4) Subsequently, the nylon bag was drawn up, hung for 15 minutes for water draining, and then centrifuged in a centrifuge at 90 G for 90 seconds for dehydration.
(5) The nylon bag containing the dehydrated water-absorbent composite therein was measured of the weight, which was defined as W3.
(6) The same fiber as used in producing the composite was similarly placed at the same weight as the weight (W2) contained in the composite, in a 250-mesh nylon bag (20 cm × 10 cm). The nylon bag with the fiber therein was dipped in 500 ml of physiological saline at room temperature for 30 minutes.
(7) Then, the nylon bag was drawn up, hung for 15 minutes for water draining, and then centrifuged in the centrifuge under 90 G for 90 seconds for dewatering. The nylon bag with the dehydrated fiber therein was measured of the weight, which was defined as W4.

(8) The water retentive capacity "S" of the composite for physiological saline was calculated according to the equation mentioned below. In this formula, W1 to W4 were all expressed in unit gram (g).

$$\texttt{Water Retentive capacity, S = [(W3 - W4)/(W1 - W2)]}$$

3-6) Water-absorbent rate

By the same method as in 3-5) above except for the modification of the dipping time in physiological saline (30 minutes) according to the measurement method above in 3-5) into 5 minutes, W1 to W4 were determined. By the same calculation formula as in 3-5), the water-absorbent rate of the water-absorbent resin composite composition (= the water retentive capacity in 5 minutes) was calculated.

4. Highly densified water-absorbent resin composite composition

4-1) Thickness

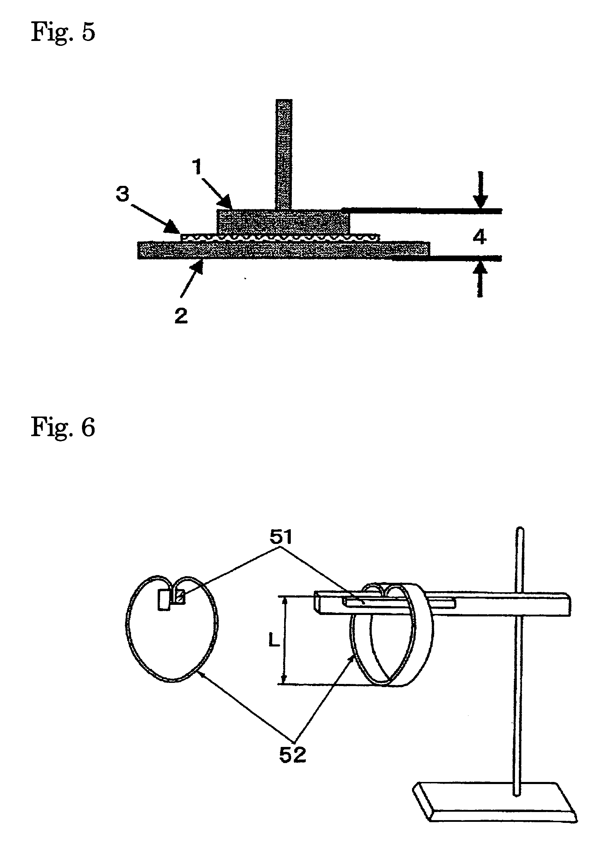

The highly densified water-absorbent resin composite composition was cut into a piece of 5 cm x 5 cm. The thickness of the highly densified water-absorbent resin composite composition was measured according to JIS 1-1096 (Fig. 5).

(1) An adapter 1 of a 30-mm diameter was fitted onto a rheometer (a product of FUDOH; Model No. NRM-2003J), to adjust a sample stand 2 to an elevation speed of 2 cm/min, until the sample stand could stop, just when the load reached a pressure of 0.2 psi.
(2) A sample 3 was placed on a table for measurement. Then, the sample stand 2 was moved up. At a position where the stand stopped just when the load reached a pressure of 0.2 psi, the distance 4 between the upper face of the adapter 1 and the lower face of the sample stand 2 was measured with a caliper.
(3) Five samples were measured, to calculate the average.
(4) With no sample on the sample stand 2, the blank measurement was carried out simultaneously.
(5) The thickness of the sample was determined according to the following equation:

$$\texttt{Thickness (mm) = sample value measured (mm) - blank value}$$
$$\texttt{measured (mm)}$$

4-2) Bulk density

The high-density water-absorbent resin composite composition was cut into a piece of 5 cm x 5 cm. The weight of the sample was measured, to calculate the bulk density thereof according to the equation mentioned below. Five samples were measured, to calculate the average.

$$\texttt{Bulk Density (g/cm}^3\texttt{) = [(sample weight in g)/(sample thickness}$$
$$\texttt{in cm) × (sample area in cm}^2\texttt{)]]}$$

4-3) Pliability

The highly densified water-absorbent resin composite composition was cut into a piece of 2 cm × 25 cm. The piece was kept at a temperature of 25°C and a humidity of 50% all through day and night. Then, the pliability thereof was measured according to the heart loop method of JIS L-1096 shown in Fig. 6, which is for use in testing relatively soft fabrics.

(1) A sample piece 52 was fitted in a heart loop form onto the gripper 51 of the horizontal bar shown in Fig. 6, to adjust the effective sample piece length to 20 cm.

(2) One minute later, the distance "L" (cm) between the top of the horizontal bar and the lowest point of the loop was measured. The L was defined as pliability. Five sample pieces were tested, to calculate the average.

Furthermore, the samples in Comparative Examples 2, 4 and 5 had no softness so that the samples could not be prepared into any heart loop form but were broken immediately. Therefore, these samples could not be measured.

4-4) Restoring ratio

The high-density water-absorbent resin composite composition was cut into a piece of 5 cm x 5 cm. The sample was compressed at a pressure of 10 MPa over 10 min. According to the method for measuring thickness in 4-2), the thickness of the sample was measured just after the compression and after storage at a temperature of 25°C and a humidity of 50% for 30 days. The restoring ratio thereof was calculated according to 50°C the following equation. Five samples were tested, to calculate the average.

```
Restoring ratio (%) = {[(thickness in mm 30 days after
compression) - (thickness in mm immediately after
compression]/(thickness in mm immediately after compression)}
× 100
```

4-5) Opening property

(1) About 5 g of the water-absorbent resin composite composition was sandwiched between a pair of hand carders (22 cm × 12.5 cm) manufactured by Ash Ford, for manual combing five times.
(2) Based on the easiness of combing and on the status of the broken water-absorbent resin particles after combing, the sample was evaluated in three grades as follows:

A: sample easily combed; almost no damage of water-absorbent resin particles after combing.
B: resistance observed against combing; damage of water-absorbent resin particles as observed after combing.
C: strong resistance against combing at a level such that combing was almost impossible, or some strong resistance against combing involving a significant damage of water-absorbent resin particles after combing.

5. Absorbent articles

5-1) Absorption rate and amount of released water

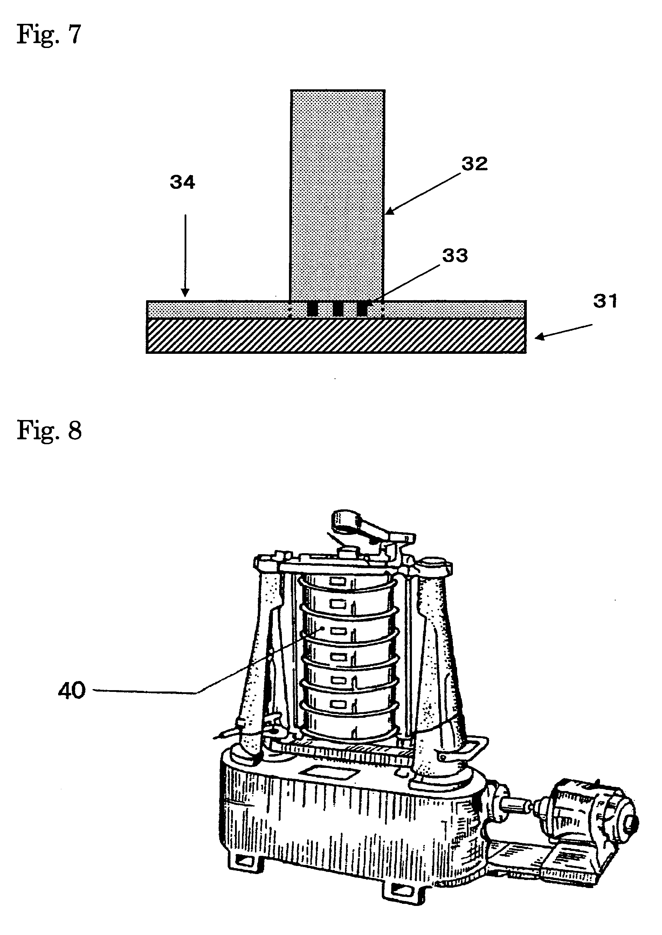

Absorption rate of artificial urine into absorbent articles and the amount of released artificial urine under pressure from the absorbent articles were measured by the following methods. The absorbent article 31 was placed on a smooth plane table. Then, an acrylic plate 34 (100 × 100 × 10 mm in total weight of 150 g) was placed as shown in Fig. 7, where a cylinder 32 of an inner diameter of 40 mm and with the upper end opened was arranged at the center and seven through-holes 33 of a diameter of 5 mm were arranged at an almost equal interval on a part enclosed with the cylinder 32. Then, a metal circular plate (500 g) of a diameter of 100 mm and with a hole of a diameter of 45 mm at the center was arranged through the cylinder 32. 25 ml of the artificial urine was placed in the cylinder 32, to measure the time required for the absorption of the urine with a stopwatch. The time was defined as absorption rate (second). 10 minutes later, the circular plate and the acrylic plate 34 with the cylinder 32 were removed. Then, 20 filters overlaid together were arranged on the same position as the position where the acrylic plate 34 was placed on the absorbent article 31. Each of the filters was a product manufactured by Toyo Filter Co. , Ltd. , under the trade name of ADVANTEC No. 424 in a size of 100 × 100 mm. Additionally, a 4-kg load with a bottom surface area (10 cm × 10cm) was placed on the filters. Five minutes later, the load was removed to measure the weight of the filters, to determine the amount of the artificial urine absorbed in the filters, which was defined as the amount of released water (g). The measurement was done triplicately. The average was defined as the amount of released water.

5-2) Water-absorbent resin dropout ratio

(1) The water-absorbent article was cut into a piece having a size of 10 cm × 10 cm (its four edges were all open). Then, the weight was measured. The overall weight of the water-absorbent resin in the sample was calculated, on the basis of the weight percent of the water-absorbent resin in the composite. Using a tape, the piece of the water-absorbent article was fixed in the center of a standard sieve defined by JIS Z8801 (its inner frame dimension was as follows: the inner diameter was 150 mm; the depth was 45 mm; and the pore size was 20 mesh).

(2) A ro-tap shaker of Tokyo Shinohara Seisakusho's Model SS-S-228 was prepared. Then, the water-absorbent article was fixed, only on the uppermost step in the figure (Fig. 8) of JIS Z8815.

(3) The shaker was set to a number of impacts being 165 impacts/min and a number of rotation being 290 rpm. 60 minutes after shaking, the weight of the water-absorbent resin particle dissociated from the water-absorbent article was measured. The dropout ratio was determined according to the following equation.

$$\texttt{Dropout ratio (\%) of water-absorbent resin = [(weight of the}$$
$$\texttt{dissociated water-absorbent resin in g)/(weight of the total}$$
$$\texttt{water-absorbent resin before shaking in g)] × 100.}$$

5-3) Gel dropout ratio

The amount of dropout water-absorbent gel in the water-absorbent article was measured by the following procedures, when a power was repeatedly loaded on the water-absorbent article to work to rub the water-absorbent article together.

(1) The water-absorbent article 31 was placed on a flat and smooth bed. An acrylic plate 34 (100 × 100 × 10 mm in total weight of 150 g) was placed as shown in Fig. 7, where a cylinder 32 of an inner diameter of 40 mm and the upper end opened was arranged at the center and seven through-holes 33 each having a diameter of 5 mm were arranged at almost an equal interval in the part enclosed by the cylinder 32.

(2) 150 ml of artificial urine (the composition is mentioned below) was poured into the cylinder to allow the water-absorbent article to absorb water.

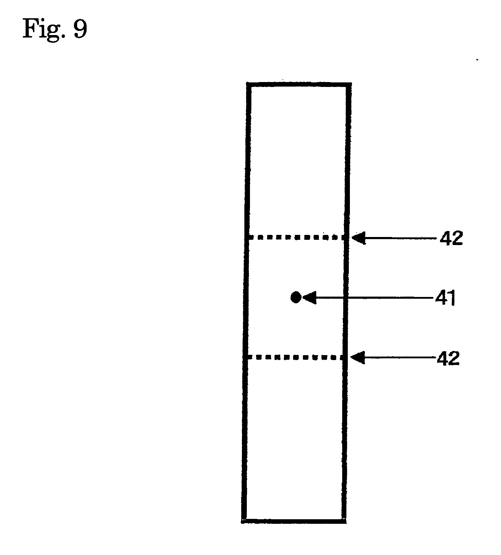

(3) After the article had fully absorbed water, it was further kept at room temperature for 30 minutes. Then, this was cut along the cutting lines 42 located 5 cm apart from the center 41, as in Fig. 9. The weight of the cut piece was measured.

(4) After the measurement, the piece was placed on the center of an acrylic plate of 20 cm × 20 cm. A load (3 kg.) with the same bottom surface area (10 cm × 10 cm) as the area of the sample cut piece was placed just on the sample piece in a manner that the load would never come out of the sample piece.

(5) Then, the integral sample was set in a shaker (Iuchi Seieido's Model MS-1) in such a manner that the cut edge of the sample was perpendicular to the running direction of the shaker. The sample was shaken at an amplitude of 50 mm and a vibration number of 80 vibrations/min, for 30 minutes.

(6) After shaking, the load was released from the sample. The weight of the water-absorbent gel having dropped off from the sample was measured. The gel dropout ratio was calculated according to the following equation.

$$\texttt{Gel dropout ratio (\%) = [(amount of dropout gel in g)/(total}$$
$$\texttt{amount of gel before the test in g)] × 100}$$

6. Others

The artificial urine of the following composition was used in 5-1) the measurement of water-absorbent rate and amount of released water and 5-3) the measurement of gel dropout ratio:

| | |
|---|---|
| Urea | 1.94 wt% |

Table continued

| Sodium chloride | 0.80 wt% |
| Calcium chloride | 0.06 wt% |
| Magnesium sulfate | 0.11 wt% |
| Distilled water | 97.09 wt% |

Table 1

| | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Comp. Ex. 1 | Comp. Ex. 2 | Comp. Ex. 3 | Comp. Ex. 4 | Comp. Ex. 5 |
|---|---|---|---|---|---|---|---|---|---|---|---|---|---|---|---|---|---|---|
| **Production conditions of water-absorbent resin composites** | | | | | | | | | | | | | | | | | | |
| Production Examples | Prod. Ex. 1 | Prod. Ex. 2 | Prod. Ex. 3 | Prod. Ex. 4 | Prod. Ex. 3 | Prod. Ex. 4 | Prod. Ex. 5 | Prod. Ex. 6 | Prod. Ex. 3 | Prod. Ex. 3 | Prod. Ex. 3 | Prod. Ex. 9 | Prod. Ex. 10 | Prod. Ex. 7 | Prod. Ex. 8 | Prod. Ex. 11 | Prod. Ex. 12 | Prod. Ex. 13 |
| Fiber materials | pulp | pulp | pulp | pulp | pulp | pulp | PET | nylon/ rayon | pulp | pulp | pulp | pulp | pulp | pulp | pulp | PET | pulp | PP/PE |
| Average fiber length [μm] | 2500 | 2500 | 2500 | 2500 | 2500 | 2500 | 900 | 900 | 2500 | 2500 | 2500 | 2500 | 2500 | 2500 | 2500 | 900 | 2500 | 2500 |
| Average fiber diameter [dtex] | 2.2 | 2.2 | 2.2 | 2.2 | 2.2 | 2.2 | 1.7 | 1.7 | 2.2 | 2.2 | 2.2 | 2.2 | 2.2 | 2.2 | 2.2 | 1.7 | 2.2 | 1.5 |
| Fiber contact angle with water [°] | 0 | 0 | 0 | 0 | 0 | 0 | 80 | 50/0 | 0 | 0 | 0 | 0 | 0 | 0 | 0 | 80 | 0 | 90 |
| Spatial fiber density [g/m³] | 200 | 80 | 40 | 10 | 40 | 40 | 40 | 40 | 40 | 40 | 40 | 60 | - | - | 40 | 60 | - | 40 |
| Diameter of liquid droplet [μm] | 430 | 450 | 450 | 450 | 450 | 450 | 440 | 450 | 450 | 450 | 450 | 500 | - | - | - | 250 | - | 500 |
| Spatial density of liquid droplets [g/m³] | 2 | 2 | 2 | 2 | 2 | 2 | 2 | 2 | 2 | 2 | 2 | 2 | - | - | 2 | 3 | - | 2 |
| Position of feed port [m] | 1.6 | 1.6 | 1.6 | 1.6 | 1.6 | 1.6 | 1.6 | 1.6 | 1.6 | 1.6 | 1.6 | 0.8 | - | - | 0.8 | 0.8 | - | 1.6 |
| Polymerization ratio at feed port [%] | 40 | 40 | 40 | 40 | 40 | 40 | 40 | 40 | 40 | 40 | 40 | 15 | - | - | 15 | <1 | - | 40 |
| Position for composite recovery [m] | 3.0 | 3.0 | 3.0 | 3.0 | 3.0 | 3.0 | 3.0 | 3.0 | 3.0 | 3.0 | 3.0 | 3.0 | - | - | 1.6 | 3.0 | - | 3.0 |
| **Conditions for procedures for high densification** | | | | | | | | | | | | | | | | | | |
| Coating weight of water-absorbent resin [g/m²] | 300 | 300 | 300 | 300 | 150 | 150 | 300 | 300 | 300 | 300 | 300 | 300 | 300 | 300 | 300 | 300 | 300 | 300 |
| Pressure [MPa] | 0.6 | 0.6 | 0.6 | 0.6 | 0.6 | 0.6 | 0.6 | 0.6 | 0.6 | 0.6 | 0.6 | 0.6 | 0.6 | 0.6 | 0.6 | 0.6 | 0.6 | 0.6 |
| Heating [°C] | room temp. | room temp. | room temp. | room temp. | room temp. | room temp. | room temp. | room temp. | 150 | room temp. | 150 | room temp. | room temp. | room temp. | room temp. | room temp. | 150 | 170 |
| Humidification | no | no | no | no | no | no | no | no | no | yes (water spraying) | yes (STM) | no | no | no | no | no | yes (STM) | no |
| **Assessment of water-absorbent resin composite and assembly** | | | | | | | | | | | | | | | | | | |
| Average particle diameter of water-absorbent resin [μm] | 400 | 400 | 400 | 400 | 400 | 400 | 400 | 400 | 400 | 400 | 400 | 400 | 400 | 600 | aspherical | 200 | 370 | 400 |
| Dry weight ratio of binding fiber and water-absorbent resin [w/w] | 70:30 | 45:55 | 30:70 | 9:91 | 30:70 | 9:91 | 30:70 | 30:70 | 30:70 | 30:70 | 30:70 | 30:70 | 30:70 | 30:70 | 30:70 | 30:70 | - | 30:70 |
| Water retentive capacity [g/g] | 45 | 45 | 44 | 47 | 45 | 45 | 45 | 45 | 45 | 45 | 45 | 45 | 45 | 30 | 32 | 37 | - | 43 |
| Water-absorbent rate [g/g] | 37 | 38 | 38 | 39 | 35 | 39 | 36 | 37 | 36 | 37 | 37 | 37 | 37 | 25 | 26 | 24 | - | 38 |

Table 1 (continued)

| | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Comp. Ex. 1 | Comp. Ex. 2 | Comp. Ex. 3 | Comp. Ex. 4 | Comp. Ex. 5 |
|---|---|---|---|---|---|---|---|---|---|---|---|---|---|---|---|---|---|---|
| Assessment of highly densified water-absorbent resin composite composition | | | | | | | | | | | | | | | | | | |
| Weight ratio of composite A | 20 | 20 | 20 | 20 | 20 | 20 | 20 | 20 | 20 | 20 | 20 | 30 | 24 | 0 | 0 | 0 | - | 0 |
| Weight ratio of composite B | 0 | 0 | 0 | 0 | 0 | 0 | 0 | 0 | 0 | 0 | 0 | 70 | 38 | 100 | 100 | 0 | - | 0 |
| Weight ratio of composite C | 80 | 80 | 80 | 80 | 80 | 80 | 80 | 80 | 80 | 80 | 80 | 0 | 33 | 0 | 0 | 100 | - | 100 |
| Free fiber | 0 | 0 | 0 | 0 | 0 | 0 | 0 | 0 | 0 | 0 | 0 | 0 | 5 | 0 | 0 | 0 | - | 0 |
| Dry weight ratio of free fiber and water-absorbent resin [w/w] | 0:100 | 0:100 | 0:100 | 0:100 | 0:100 | 0:100 | 0:100 | 0:100 | 0:100 | 0:100 | 0:100 | 0:100 | 5.5:94.5 | 0:100 | 0:100 | 0:100 | 30:70 | 0:100 |
| Average fiber length of free fiber [μm] | - | - | - | - | - | - | - | - | - | - | - | - | 2500 | - | - | - | 2500 | - |
| Thickness [mm] | 4.0 | 1.5 | 1.3 | 0.8 | 1.0 | 0.5 | 2.0 | 2.0 | 0.8 | 0.9 | 0.7 | 1.3 | 1.5 | 2.0 | 3.0 | 2.0 | 3.5 | 3.5 |
| Bulk density [g/cm³] | 0.25 | 0.36 | 0.33 | 0.41 | 0.21 | 0.33 | 0.21 | 0.21 | 0.54 | 0.48 | 0.61 | 0.33 | 0.29 | 0.21 | 0.14 | 0.21 | 0.00 | 0.12 |
| Pliability [cm] | 9.5 | 9.0 | 9.0 | 8.5 | 9.0 | 8.5 | 9.0 | 9.0 | 8.5 | 9.0 | 8.5 | 8.5 | 9.0 | 9.0 | cannot be measured | 9.0 | cannot be measured | cannot be measured |
| Restoring ratio [%] | 25 | 22 | 9 | 11 | 13 | 15 | 40 | 40 | 20 | 22 | 22 | 9 | 13 | 25 | 35 | 40 | 95 | 15 |
| Opening property | O | O | O | O | O | O | O | O | O | O | O | O | O | × | × | × | × | × |
| Assessment of water-absorbent article | | | | | | | | | | | | | | | | | | |
| Absorption rate [sec] | 5 | 5 | 6 | 6 | 6 | 8 | 3 | 3 | 6 | 6 | 7 | 7 | 6 | 6 | 7 | 7 | 5 | 5 |
| Amount of released water [g] | 3.0 | 2.5 | 1.9 | 1.5 | 2.3 | 2.0 | 1.5 | 1.5 | 2.3 | 2.0 | 2.4 | 2.0 | 2.5 | 6.7 | 8.9 | 10.0 | 1.9 | 4.2 |
| Dropout ratio of water-absorbent rein [%] | 0.0 | 0.0 | 0.5 | 0.9 | 0.0 | 1.2 | 0.0 | 0.0 | 0.0 | 0.0 | 0.0 | 1.2 | 0.5 | 6.0 | 9.0 | 22.0 | 22.5 | 0.3 |
| Gel dropout ratio [%] | 0.5 | 0.5 | 1.5 | 1.8 | 0.4 | 1.1 | 2.0 | 3.0 | 0.5 | 0.5 | 0.5 | 1.5 | 1.5 | 13.0 | 15.0 | 17.0 | 18.7 | 5.0 |

INDUSTRIAL APPLICABILITY OF THE INVENTION

The absorbent article in accordance with the invention is preferably used in sanitary goods such as paper diapers and sanitary napkins, industrial materials required for the absorption and retention of liquid waste and the like, and other agricultural materials such as freshness keepers of vegetables and the like and water retentive materials. Additionally, the method for producing the absorbent article in accordance with the invention can be practiced using an industrial production system. Thus, the method is great for mass-scale production. Therefore, the invention has high industrial applicability.

**Claims**

1.  An absorbent article containing a water-absorbent resin composite wherein
    the water-absorbent resin composite comprises one water-absorbent resin particle and two or more fibers,
    the water-absorbent resin particle is nearly spherical,
    one or more of the two or more fibers are partially embedded in the resin particle and are partially exposed from the resin particle, and
    one or more of the two or more fibers are never embedded in the resin particle but partially adhere to the surface of the resin particle.

2.  The absorbent article according to claim 1, wherein the weight of the water-absorbent resin particle dissociated from the water-absorbent article is at 5% by weight or less of the total weight of the water-absorbent resin in the water-absorbent article before shaking, when the water-absorbent article is shaken at a vibration number of 165 vibrations/ minute and a rotation number of 290 rotations/minute for 60 minutes, using a shaker shown in the Fig. 4 of JIS Z-8815.

3.  The absorbent article according to claim 1, which comprises a water-absorbent resin composite composition with a restoring ratio of 50% or less, as represented by the following formula:

$$\text{restoring ratio (\%)} = (A-B)/B \times 100 \qquad (1)$$

    wherein A represents the thickness of the water-absorbent resin composite composition after compressing the water-absorbent resin composite composition at a pressure of 100 Kg/cm$^2$ for 10 minutes and then storing the water-absorbent resin composite composition at a temperature of 25°C, atmospheric pressure and a humidity of 50% for 30 days; and B represents the thickness of the water-absorbent resin composite composition immediately after compressing the water-absorbent resin composite composition at a pressure of 100 Kg/cm$^2$ for 10 minutes.

4.  The absorbent article according to claim 1, which has a gel dropout ratio represented by the following formula (2) of 10% or less:

$$\text{gel dropout ratio (\%)} = A/B \times 100 \qquad (2)$$

    wherein A represents the weight of a dropout water-absorbent gel after mounting a load of 3 Kg on the absorbent article after water absorption and shaking the absorbent article with an amplitude of 50 cm and the number of vibration being 80 vibrations/ minute for 30 minutes; and B represents the weight of the water-absorbent gel before shaking.

5.  The absorbent article according to claim 1, which comprises a water-absorbent resin composite composition with a pliability of 5.0 to 9.5 cm, as determined by the heart loop method defined according to JIS L-1096.

6.  An absorbent article containing a water-absorbent resin particle and a fiber and comprising a water-absorbent resin composite composition with a restoring ratio of 50% or less, as represented by the following formula:

$$\text{restoring ratio (\%)} = (A-B)/B \times 100 \qquad (1)$$

wherein A represents the thickness of the water-absorbent resin composite composition after compressing the water-absorbent resin composite composition at a pressure of 100 Kg/cm$^2$ for 10 minutes and then storing the water-absorbent resin composite composition at a temperature of 25°C, atmospheric pressure and a humidity of 50% for 30 days; and B represents the thickness of the water-absorbent resin composite composition immediately after compressing the water-absorbent resin composite composition at a pressure of 100 Kg/cm$^2$ for 10 minutes.

7. An absorbent article containing a water-absorbent resin particle and a fiber and comprising a water-absorbent resin composite composition with a gel dropout ratio of 10% or less as represented by the following formula (2):

$$\texttt{gel dropout ratio (\%) = A/B × 100} \qquad (2)$$

wherein A represents the weight of a dropout water-absorbent gel after mounting a load of 3 Kg on the absorbent article after water absorption and shaking the absorbent article with an amplitude of 50 cm and the number of vibration being 80 vibrations/ minute for 30 minutes; and B represents the weight of the water-absorbent gel before shaking.

8. An absorbent article containing a water-absorbent resin particle and a fiber and comprising a water-absorbent resin composite composition with a pliability of 5.0 to 9.5 cm, as determined by the heart loop method defined according to JIS L-1096.

9. The absorbent article according to any one of claims 1 to 8, wherein the dry weight ratio of the fiber and the water-absorbent resin particle is 1:1 to 1:1,000,000.

10. The absorbent article according to any one of claims 1 to 9, wherein the average particle size of the water-absorbent resin particle is 50 to 1,000 $\mu$m.

11. The absorbent article according to any one of claims 1 to 10, wherein the average fiber length of the fiber is 50 to 50,000 $\mu$m.

12. The absorbent article according to any one of claims 1 to 11, wherein the average fiber diameter is 0.1 to 500 dtex.

13. The absorbent article according to any one of claims 1 to 12, wherein one or more of the two or more fibers are a fiber with a contact angle with water being 60° or less.

14. The absorbent article according to claim 13, wherein the fibers are cellulose.

15. The absorbent article according to any one of claims 1 to 14, wherein the water-absorbent resin is a crosslinked unsaturated carboxylic acid polymer.

16. The absorbent article according to any one of claims 1 to 15, wherein the absorbent article consisting mainly of a water-absorbent resin and a fiber and comprises a water-absorbent resin composite composition comprising the water-absorbent resin composite.

17. The absorbent article according to claim 16, which comprises the water-absorbent resin composite at a weight fraction ratio of 0.1 or more in the water-absorbent resin composite composition.

18. The absorbent article according to claim 16 or 17, wherein the absorbent resin contains a water-absorbent resin composite comprising one or more water-absorbent resin particles and one or more fibers in the water-absorbent resin composite composition, wherein the water-absorbent resin particles are nearly spherical and the one or more fibers are partially embedded in the resin particles and are partially exposed from the resin particles and wherein none of the fibers adheres to the surface of the resin particles.

19. The absorbent article according to any one of claims 16 to 18, wherein the absorbent resin contains a water-absorbent resin composite comprising one or more water-absorbent resin particles and one or more fibers in the water-absorbent resin composite composition, wherein the water-absorbent resin particles are nearly spherical and the one or more fibers partially adhere to the surface of the resin particles and none of the fibers is embedded in the resin particles.

**20.** The absorbent article according to any one of claims 16 to 19, wherein the absorbent article comprises one or more fibers never embedded in the water-absorbent resin or never adhering to the water-absorbent resin in the water-absorbent resin composite composition.

**21.** The absorbent article according to claim 20, wherein the fiber never embedded in or adhering to the water-absorbent resin and the water-absorbent resin in the water-absorbent resin composite composition are at a dry weight ratio of 90:10 to 5:95.

**22.** The absorbent article according to claim 20 or 21, wherein the length of the fiber never embedded in or adhering to the water-absorbent resin is 50 to 50,000 $\mu$m.

**23.** The absorbent article according to any one of claims 16 to 22, wherein the bulk density of the water-absorbent resin composite composition is 0.20 to 1.10 g/cm$^3$.

**24.** The absorbent article according to any one of claims 16 to 23, wherein the thickness of the water-absorbent resin composite composition is 0.2 to 5.0 mm.

**25.** The absorbent article according to any one of claims 16 to 24, wherein the water-absorbent resin composite composition can be opened.

**26.** A sanitary material using an absorbent article according to any one of claims 1 to 26.

**27.** An industrial material using an absorbent article according to any one of claims 1 to 25.

**28.** An agricultural material using an absorbent article according to any one of claims 1 to 25.

**29.** A method for producing an absorbent article according to any one of claims 1 to 5, comprising pressurizing a water-absorbent resin composite composition comprising the water-absorbent resin composite.

**30.** The method for producing an absorbent article according to claim 29, comprising heating the water-absorbent resin composite composition at a temperature lower than the melting temperature of the fiber.

**31.** The method for producing an absorbent article according to claim 29 or 30, wherein pressurizing the water-absorbent resin composite composition is conducted under humidification.

Fig. 1

Fig. 2

Fig. 3

Fig. 4

Fig. 5

Fig. 6

Fig. 7

Fig. 8

Fig. 9

<table>
<tr><td colspan="3" align="center">**INTERNATIONAL SEARCH REPORT**</td><td>International application No.<br><br>PCT/JP2004/008177</td></tr>
</table>

| | |
|---|---|
| A. CLASSIFICATION OF SUBJECT MATTER<br>    Int.Cl⁷ B01J20/26, B32B5/30 | |

A. CLASSIFICATION OF SUBJECT MATTER
    Int.Cl$^7$ B01J20/26, B32B5/30

According to International Patent Classification (IPC) or to both national classification and IPC

B. FIELDS SEARCHED

Minimum documentation searched (classification system followed by classification symbols)
    Int.Cl$^7$ B01J20/00-20/34, B32B1/00-35/00

Documentation searched other than minimum documentation to the extent that such documents are included in the fields searched

| | | | |
|---|---|---|---|
| Jitsuyo Shinan Koho | 1926–1996 | Toroku Jitsuyo Shinan Koho | 1994–2004 |
| Kokai Jitsuyo Shinan Koho | 1971–2004 | Jitsuyo Shinan Toroku Koho | 1996–2004 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2003-11118 A  (Mitsubishi Chemical Corp.),<br>15 January, 2003 (15.01.03),<br>(Family: none) | 1,2,4,7,9-31<br>3,5,6,8 |
| X<br>A | JP 2003-10680 A  (Mitsubishi Chemical Corp.),<br>14 January, 2003 (14.01.03),<br>(Family: none) | 1,2,4,7,9-31<br>3,5,6,8 |
| X<br>A | JP 2002-361079 A  (Mitsubishi Chemical Corp.),<br>17 December, 2002 (17.12.02),<br>(Family: none) | 1,2,4,7,9-31<br>3,5,6,8 |
| X<br>A | JP 2002-370025 A  (Mitsubishi Chemical Corp.),<br>24 December, 2002 (24.12.02),<br>(Family: none) | 1,2,4,7,9-31<br>3,5,6,8 |

| | | |
|---|---|---|
| ☒  Further documents are listed in the continuation of Box C. | ☐  See patent family annex. | |

| | | | |
|---|---|---|---|
| *<br>"A"<br><br>"E"<br><br>"L"<br><br><br>"O"<br>"P" | Special categories of cited documents:<br>document defining the general state of the art which is not considered to be of particular relevance<br>earlier application or patent but published on or after the international filing date<br>document which may throw doubts on priority claim(s) or which is cited to establish the publication date of another citation or other special reason (as specified)<br>document referring to an oral disclosure, use, exhibition or other means<br>document published prior to the international filing date but later than the priority date claimed | "T"<br><br><br>"X"<br><br><br><br>"Y"<br><br><br><br>"&" | later document published after the international filing date or priority date and not in conflict with the application but cited to understand the principle or theory underlying the invention<br>document of particular relevance; the claimed invention cannot be considered novel or cannot be considered to involve an inventive step when the document is taken alone<br>document of particular relevance; the claimed invention cannot be considered to involve an inventive step when the document is combined with one or more other such documents, such combination being obvious to a person skilled in the art<br>document member of the same patent family |

| Date of the actual completion of the international search<br>    27 August, 2004 (27.08.04) | Date of mailing of the international search report<br>    21 September, 2004 (21.09.04) |
|---|---|
| Name and mailing address of the ISA/<br>    Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2004/008177

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2000-328456 A (Mitsubishi Chemical Corp.),<br>28 November, 2000 (28.11.00),<br>& WO 00/55418 A          & EP 1178149 A<br>& US 2002/53754 A | 1,2,4,7,9-31<br>3,5,6,8 |
| X<br>A | JP 9-67403 A (Mitsubishi Chemical Corp.),<br>11 March, 1997 (11.03.97),<br>(Family: none) | 1,2,4,7,9-31<br>3,5,6,8 |
| X<br>A | JP 11-93073 A (Kao Corp.),<br>06 April, 1999 (06.04.99),<br>(Family: none) | 1,2,4,7,9-31<br>3,5,6,8 |

Form PCT/ISA/210 (continuation of second sheet) (January 2004)

<div align="center"><b>INTERNATIONAL SEARCH REPORT</b></div>

| International application No. |
| --- |
| PCT/JP2004/008177 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

The matter common to claims 1, 6, 7, and 8 is only an absorbent article containing water-absorbing resin particles and fibers, but such absorbent articles are well known as disclosed in documents cited in the description of this application, "Background art" (pp.1-2). Thus, the absorbent article lacks novelty and is not considered to be "a special technical feature" within the meaning of PCT Rule 13.2, second sentence. Further, the other invention-specifying matters of claims 1, 6, 7, and 8 are entirely different from each other.
   Accordingly, there is no special technical feature common to claims 1, (continued to extra sheet.)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.  Consequently, this international search report is restricted to the invention first mentioned in the claims; it is covered by claims Nos.:

**Remark on Protest**        ☐  The additional search fees were accompanied by the applicant's protest.

☒  No protest accompanied the payment of additional search fees.

Form PCT/ISA/210 (continuation of first sheet (2)) (January 2004)

| INTERNATIONAL SEARCH REPORT | International application No. |
| --- | --- |
| | PCT/JP2004/008177 |

<u>Continuation of Box No.III of continuation of first sheet(2)</u>
6, 7, and 8, and the inventions of these claims do not comply with the requirement of unity of invention.

Therefore, the number of inventions of this international application is 4, that is, claims 1-5 and 9-31, claim 6, claim 7, and claim 8.

Claim 1

The absorbent article of claim 1 is one made by using a water-absorbing composite (hereinafter referred to as "composite A") comprising one nearly spherical water-absorbing resin particle and two or more fibers, at least one of which fibers is partially embedded in the resin particle and partially exposed and at least another of which fibers is partially bonded to the surface of the resin particle without being embedded in the resin particle. However, the description discloses only absorbent articles made by using "a high-density water absorbent composite composition" (see the description, p. 43, lines 20ff.) which is produced by pressing a mixture containing a great number of composites A under the prescribed conditions, but does not disclose any absorbent article made by using only one composite A consisting of one resin particle and two fibers. Further, such an absorbent article is not considered at all as serving practically as an absorbent article.

Claims 6-8

As to "a water-absorbent resin composite composition having a resilience of 50 % or below" as set forth in claim 6 and "a water-absorbent resin composite composition having a bending resistance of 5.0 to 9.5 cm" as set forth in claim 8, only "high-density water-absorbent composite compositions" are disclosed in the description. Thus, neither what compositions the "water-absorbent resin composite composition having a resilience of 50 % or below" or the "water-absorbent resin composite composition having a bending resistance of 5.0 to 9.5 cm" includes nor how such compositions can be produced can be imagined. Further, it cannot be considered that other compositions exhibit action and effect equivalent to those of the high-density water-absorbent composite compositions so long as they satisfy the requirements of resilience or bending resistance.

As to "an absorbent article having a fall-off of gel of 10 % or below" as set forth in claim 7, only use of the "high-density water-absorbent composite composition" is disclosed as the mean for attaining such a fall-off of gel.

Among the inventions of claims 1 and 6-8 and claims referring to them, as described above, only articles made by using the "high-density water-absorbent composite composition" are disclosed within the meaning of PCT Article 5, and therefore this search has been made on water absorbent articles made by using the "high-density water-absorbent composite composition".

Additionally, as to the determination of resilience, bending resistance, and fall-off of gel, only methods for the "high-density water-absorbent composite composition" are disclosed. Therefore, e.g., how the resilience of an article made by using a composite constituted of one resin particle and one fiber is determined cannot be understood at all. Thus, the scope of articles having resilience, bending resistance and fall-off of gel as set forth in claims 6-8 cannot be specified, and the claims 6-8 lacks also the requirement of clearness of PCT Article 6.

Form PCT/ISA/210 (extra sheet) (January 2004)